(19) Europäisches Patentamt
European Patent Office
Office européen des brevets

(11) Veröffentlichungsnummer: **0 007 067**
**A1**

(12) # EUROPÄISCHE PATENTANMELDUNG

(21) Anmeldenummer: 79102272.6

(22) Anmeldetag: 04.07.79

(51) Int. Cl.³: C 07 D 211/14
C 07 D 211/22, C 07 D 211/32
C 07 D 211/70, A 61 K 31/445

(30) Priorität: 05.07.78 CH 7332/78

(43) Veröffentlichungstag der Anmeldung:
23.01.80 Patentblatt 80/2

(84) Benannte Vertragsstaaten:
AT BE CH DE FR GB IT LU NL SE

(71) Anmelder: CIBA-GEIGY AG
Patentabteilung Postfach
CH-4002 Basel(CH)

(72) Erfinder: Haas, Georges, Dr.
Im Rehwechsel 24
D-4102 Binningen(CH)

(72) Erfinder: Rossi, Alberto, Dr.
Bündtenweg 30
D-4104 Oberwil(CH)

(72) Erfinder: Ferrini, Pier Giorgio, Dr.
Im Rehwechsel 22
D-4102 Binningen(CH)

(72) Erfinder: Schier, Oswald, Dr.
Amselstrasse 15
D-4104 Oberwil(CH)

(74) Vertreter: Zumstein sen., Fritz, Dr. et al,
Bräuhausstrasse 4
D-8000 München 2(DE)

(54) **Neue Phenylpiperidinderivate, Verfahren zu ihrer Herstellung, diese enthaltende pharmazeutische Präparate und ihre Verwendung.**

(57) Substituierte, gegebenenfalls einfach ungesättigte 1-Benzyl-phenylpiperidine der Formel

$$R_1 - Ph - R_2 \qquad (I),$$

worin $R_1$ einen in 1-Stellung durch gegebenenfalls substituiertes Benzyl substituierten, gegebenenfalls einfach ungesättigten Piperidylrest bedeutet, Ph einen gegebenenfalls substituierten Phenylenrest darstellt und $R_2$ einen gegebenenfalls substituierten Niederalkylrest bedeutet, und ihre pharmazeutisch verwendbaren Salze haben antithrombotische Eigenschaften. Sie können als Arzneimittel dienen und als Pharmazeutika verwendet werden.

EP 0 007 067 A1

Croydon Printing Company Ltd.

CIBA-GEIGY AG
Basel (Schweiz) 4-11901/+

Neue Phenylpiperidinderivate, Verfahren zu ihrer Herstellung, diese enthaltende pharmazeutische Präparate und ihre Verwendung.

Die Erfindung betrifft Phenylpiperidinderivate, insbesondere substituierte, gegebenenfalls einfach ungesättigte 1-Benzyl-phenylpiperidine der Formel

$$R_1 - Ph - R_2 \qquad (I),$$

worin $R_1$ einen in 1-Stellung durch gegebenenfalls substituiertes Benzyl substituierten, gegebenenfalls einfach ungesättigten Piperidylrest bedeutet, Ph einen gegebenenfalls substituierten Phenylenrest darstellt und $R_2$ einen gegebenenfalls substituierten Niederalkylrest bedeutet, und ihre pharmazeutisch verwendbaren Salze als Arzneimittel, ihre Verwendung als Pharmazeutika und die genannten Verbindungen selbst, mit der Massgabe, dass Ph gegebenenfalls substituiertes 1,2- oder 1,3-Phenylen bedeutet, wenn $R_2$ in α-Stellung eine Hydroxy-, Hydroxymethyl-, Niederalkanoyl-, 2-Oxoniederalkanoyl- oder 2-Hydroxyniederalkanoylgruppe aufweist, und mit der weiteren Massgabe dass $R_1$ von 4-(1-Benzyl)-piperidyl und 4-(1-Benzyl)-, 4-[1-(Monohalogenbenzyl)]- und 4-[1-(Monoalkylbenzyl)]-1,2,5,6-tetrahydro-pyridyl verschieden ist, wenn Ph gegebenenfalls substituiertes 1,4-Phenylen darstellt und $R_2$ Niederalkyl bedeutet oder Niederalkanoyl oder 1-Hydroxyniederalkyl mit mindestens 2 C-Atomen ist, und ihre Salze, sowie Verfahren zu ihrer Herstellung.

Der in 1-Stellung durch gegebenenfalls substituiertes Benzyl substituierte, gegebenenfalls einfach ungesättigte Piperidylrest ist beispielsweise ein entsprechender Piperidyl- oder in zweiter Linie 1,2,5,6-Tetrahydropyridylrest. Als Substituenten desselben kommen beispielsweise Niederalkyl, Niederalkoxy, Niederalkylthio, Hydroxy, Halogen und/oder Trifluormethyl, wobei einer oder mehrere der genannten Substituenten, insbesondere zwei Halogenatome, vorhanden sein können.

Der gegebenenfalls substituierte Phenylenrest ist beispielsweise ein gegebenenfalls durch Niederalkyl, Niederalkoxy, Hydroxy und/oder Halogen substituierter 1,4- oder in zweiter Linie 1,3-Phenylenrest, wobei in substituierten Phenylenresten ein, vorzugsweise in o-Stellung zu $R_1$ gebundener, oder in zweiter Linie mehr als ein Substituenten vorhanden sein kann.

Ein gegebenenfalls substituierter Niederalkylrest ist beispielsweise ein durch Oxo oder Hydroxy substituierter Niederalkylrest, vorzugsweise unsubstituiertes oder durch Hydroxy oder in zweiter Linie Oxo einfach substituiertes Niederalkyl, d.h. Niederalkyl, Hydroxyniederalkyl, vor allem α-Hydroxyniederalkyl, oder Oxoniederalkyl, vor allem Niederalkanoyl.

Vor- und nachstehend werden unter "niederen" Reste und organischen Verbindungen vorzugsweise solche verstanden, die bis und mit 7 vor allem bis und mit 4, C-Atome enthalten.

Niederalkyl ist beispielsweise Methyl, Aethyl, Propyl, Isopropyl, n-, iso-, sec.- oder tert.-Butyl, ferner n-Pentyl, n-Hexyl oder n-Heptyl.

Niederalkoxy ist beispielsweise Methoxy, Aethoxy, Propyloxy, Isopropyloxy oder n-Butyloxy.

Niederalkylthio ist beispielsweise Methylthio, Aethylthio, Propylthio, Isopropylthio oder n-Butylthio.

Hydroxyniederalkyl ist beispielsweise 2-Hydroxyäthyl, 2- oder 3-Hydroxypropyl, 4-Hydroxybutyl oder vor allem α-Hydroxyniederalkyl, wie Hydroxymethyl, 1-Hydroxyäthyl, 1-Hydroxypropyl oder 1-Hydroxybutyl, ferner 1-Hydroxypentyl, -hexyl oder -heptyl.

Oxoniederalkyl ist beispielsweise 2-Oxoäthyl, 2- oder 3-Oxopropyl, 4-Oxobutyl oder vor allem Niederalkanoyl, Formyl, Acetyl, Propionyl, Butyryl, Isobutyryl oder ferner Caproyl oder Oenanthyl.

Halogen ist beispielsweise Halogen bis Atomnummer 35, wie Chlor oder in zweiter Linie Brom oder Fluor.

Salze von Verbindungen der Formel I sind beispielsweise deren Säureadditionssalze, vorzugsweise pharmazeutisch verwendbare Säureadditionssalze, mit pharmakologisch unbedenklichen Mineral-,Sulfon- oder Carbonsäuren, wie Hydrohalogenide, z.B. Hydrochloride oder -bromide, Hydrogensulfate, Sulfonate, z.B. Benzol-, p-Toluol- oder Methansulfonate, Sulfaminate, z.B. Cyclohexylsulfaminate, oder Carbonsäuresalze, z.B. Acetate, Fumarate, Maleinate oder Tartrate, derselben.

Die Erfindung betrifft in erster Linie Verbindungen der Formel I, worin $R_1$ einen in 1-Stellung durch einen gegebenenfalls durch Niederalkyl, Niederalkoxy, Hydroxy, Halogen und/oder Chlor substituierten Benzylrest, substituierten, gegebenenfalls einfach ungesättigten Piperidylrest bedeutet, Ph einen gegebenenfalls durch Niederalkyl, Niederalkoxy, Hydroxy

und/oder Halogen substituierten Phenylenrest bedeutet und $R_2$ einen gegebenenfalls in 1-Stellung oder in höherer als der 2-Stellung durch Oxo oder in 1-Stellung durch Hydroxy substituierten Niederalkylrest bedeutet, als Arzneimittel, diese enthaltende pharmazeutische Präparate und die genannten Verbindungen selbst,mit der Massgabe, dass $R_1$ von 4-(1-Benzyl)-piperidyl und 4-(1-Benzyl)-, 4-[1-(Monoalkylbenzyl)]- und 4-[1-(Monohalogenbenzyl)]-1,2,5,6-tetrahydro-pyridyl verschieden ist, wenn Ph gegebenenfalls substituiertes 1,4-Phenylen und $R_2$ Niederalkyl ist oder Niederalkanoyl mit mindestens 2 C-Atomen darstellt.

Die Erfindung betrifft vor allem Verbindungen der Formel I, worin $R_1$ einen im Phenylteil gegebenenfalls durch Niederalkyl mit bis und mit 4 C-Atomen, wie Methyl, Niederalkoxy mit bis und mit 4 C-Atomen, wie Methoxy, Hydroxy, Halogen mit Atomnummer bis und mit 35, wie Chlor, Hydroxy und/oder Trifluormethyl substituiertes 3- oder 4-(1-Benzyl)-piperidyl- bzw. 3- oder 4-(1-Benzyl-1,2,5,6-tetrahydro)-pyridylrest bedeutet, Ph gegebenenfalls, insbesondere in o-Stellung zu $R_1$, durch Niederalkyl mit bis und mit 4 C-Atomen, wie Methyl, Niederalkoxy mit bis und mit 4 C-Atomen, wie Methoxy, Hydroxy oder Halogen mit Atomnummer bis und mit 35, wie Chlor, substituiertes 1,4- oder in zweiter Linie 1,3-Phenylen darstellt, und $R_2$ gegegebenenfalls in höherer als der 2-Stellung durch Hydroxy oder in 1-Stellung oder in höherer als der 2-Stellung durch Oxo substituiertes Niederalkyl mit bis und mit 4 C-Atomen, wie Methyl, Aethyl, 3-Hydroxypropyl, 4-Hydroxypropyl, Formyl oder Acetyl, bedeutet, als Arzneimittel, diese enthaltende pharmazeutische Präparate und die genannten Verbindungen selbst, mit der Massgabe, dass $R_1$ von unsubstituierten 4-(1-Benzyl)-piperidyl und gegebenenfalls durch Niederalkyl oder Halogen monosubstituiertem 4-(1-Benzyl-1,2,5,6-tetrahydro)-pyridyl oder Ph von gegebenenfalls substituiertem 1,4-Phenylen verschieden ist, wenn $R_2$ Nieder-

alkyl ist oder Niederalkanoyl mit mindestens 2 C-Atomen bedeutet.

Die Erfindung betrifft vorzugsweise Verbindungen der Formel I, worin $R_1$ im Phenylteil durch Niederalkoxy mit bis und mit 4 C-Atomen, wie Methoxy, und/oder Halogen mit Atomnummer bis und mit 35, wie Chlor, mono- oder disubstituiertes 3- oder vor allem 4-(1-Benzyl)-piperidyl bedeutet, Ph 1,4-Phenylen bedeutet und $R_2$ gegebenenfalls in 1-Stellung oder in höherer als der 2-Stellung durch Oxo oder in höherer als der 2-Stellung durch Hydroxy monosubstituiertes Niederalkyl mit bis und mit 4 C-Atomen, z.B. Aethyl, 3-Hydroxypropyl oder Acetyl, bedeutet, und ihre pharmazeutisch verwendbaren Säureadditionssalze.

Die Erfindung betrifft in allererster Linie Verbindungen der Formel I, worin $R_1$ im Phenylteil durch Niederalkoxy mit bis und mit 4 C-Atomen, wie Methoxy, monosubstituiertes oder durch Halogen bis und mit Atomnummer 35, wie Chlor, disubstituiertes 4-(1-Benzyl)-piperidyl bedeutet, Ph 1,4-Phenylen darstellt und $R_2$ Niederalkyl mit bis und mit 4 C-Atomen, wie Aethyl, bedeutet, und ihre pharmazeutisch verwendbaren Säureadditionssalze.

Die Erfindung betrifft namentlich die in den Beispielen genannten Verbindungen der Formel I in freier Form oder in Form pharmazeutisch verwendbarer Säureadditionssalze.

Die neuen Verbindungen können nach an sich bekannter Methode hergestellt werden, beispielsweise, indem man in einer Verbindung der Formel

$$R_A - Ph - R_2 \qquad (II),$$

worin $R_A$ einen in 5-Stellung durch im Phenylteil gegebenenfalls substituiertes Benzylamino substituierten, in 1-Stellung einen austauschbaren Rest X oder in 1,2-Stellung eine gegebenenfalls zusätzliche Doppelbindung aufweisenden, gegebenenfalls einfach ungesättigten n-Pentylrest bedeutet, oder in einem Salz davon, $R_A$ zu dem entsprechenden Rest $R_1$ ringschliesst und gewünschtenfalls ein erhaltenes Stereoisomerengemisch in die Komponenten auftrennt, die erhaltene Verbindung in eine andere Verbindung der Formel I überführt und/oder eine erhaltene freie Verbindung in ein Salz oder ein erhaltenes Salz in die freie Verbindung oder in ein anderes Salz umwandelt.

Austauschbare Reste sind dabei beispielsweise gegebenenfalls veresterte oder verätherte Hydroxygruppen oder gegebenenfalls substituierte Mercapto- oder Aminogruppen.

Veresterte Hydroxygruppen sind beispielsweise reaktionsfähige veresterte Hydroxygruppen, vor allem mit Mineralsäuren oder mit organischen Sulfonsäuren veresterte Hydroxygruppen, wie Halogen, z.B. Chlor, Brom oder Jod, Fluorsulfonyloxy, oder aromatisches oder aliphatisches Sulfonyloxy, z.B. Methan-, Aethan-, Aethensulfonyloxy, Benzol-, p-Toluol- oder p-Brombenzolsulfonyloxy. Als veresterte Hydroxygruppen kommen ferner mit organischen Säuren veresterte Hydroxygruppen, wie Niederalkanoyloxy oder gegebenenfalls, z.B. durch Halogen und/oder Nitro, substituiertes Benzoyloxy, in Betracht.

Verätherte Hydroxygruppen sind beispielsweise mit aliphatischen Alkoholen oder mit gegebenenfalls substituierten Phenolen verätherte Hydroxygruppen, wie Niederalkoxy oder gegebenenfalls durch Halogen und/oder Nitro substituierte Phenoxygruppen.

Substituierte Mercaptogruppen sind beispielsweise Aliphatylthiogruppen oder gegebenenfalls substituierte Phenylthiogruppen, wie Niederalkylthio oder gegebenenfalls durch Halogen und/oder Nitro substituierte Phenylthio oder Benzylthio. Als weitere substituierte

Mercaptogruppen kommen entsprechende Sulfoniumester, wie Diniederalkylsulfonium oder gegebenenfalls durch Halogen und/oder Nitro substituiertes Diphenylsulfonium in Betracht.

Substituierte Aminogruppen sind beispielsweise aliphatische und/oder durch gegebenenfalls substituiertes Phenyl oder Benzyl substituierte Aminogruppen, wie Mono- oder Diniederalkylamino oder gegebenenfalls durch Niederalkyl, Niederalkoxy, Hydroxy, Halogen und/oder Trifluormethyl substituierte Anilino-, Benzylamino- oder Diphenylaminogruppen. Als weitere substituierte Aminogruppen kommen entsprechende quaternäre Ammoniumgruppen, wie Triniederalkylammonium- oder Diniederalkylaminoxidgruppierungen in Betracht.

Salze von Verbindungen der Formel II sind beispielsweise deren Säureadditionssalze, vorzugsweise mineralsaure Säureadditionssalze, wie Hydrohalogenide, z.B. Hydrochloride, Hydrobromide oder Hydrogensulfate, derselben.

Die Cyclisierung des Restes $R_A$ erfolgt in üblicher Weise beispielsweise thermisch, z.B. durch Erhitzen auf etwa 50 bis 250°C, erforderlichenfalls in Gegenwart eines Kondensationsmittels und/oder wasserbindenden Mittels, in einem inerten Lösungsmittel und/oder unter Inertgas, wie Stickstoff.

Als Kondensationsmittel kommen dabei beispielsweise ausgehend von Verbindungen der Formel II, worin X gegebenenfalls verestertes Hydroxy oder eine gegebenenfalls substituierte Mercapto- oder Aminogruppe bedeutet, insbesondere basische Kondensationsmittel, wie Hydride, Alkoholate oder Amide von Alkalimetallen, z.B. Natriumhydrid, Alkalimetallniederalkanolate, wie Natriummethanolat oder Natriumäthanolat, oder Alkalimetallamide, wie Natriumamid oder Lithiumdiisopropylamid, ferner quater-

näre Ammoniumbasen, wie Benzyltriäthylammoniumhydroxid, oder auch tertiäre organische Stickstoffbasen, z.B. Pyridin oder Triäthylamin, in Betracht. Für die Cyclisierung von Verbindungen der Formel II, worin X gegebenenfalls veräthertes Hydroxy bedeutet, verwendet man beispielsweise saure Kondensationsmittel, wie Mineralsäuren, z.B. Schwefelsäure oder Phosphorsäure, oder saure Ionenaustauscher, und für die Cyclisierung von Verbindungen, in denen $R_A$ eine 1,2-Doppelbindung aufweist, beispielsweise Lewissäuren, wie Bortrifluorid oder Zinkchlorid.

Geeignete Lösungsmittel sind beispielsweise unter den Reaktionsbedingungen inerte, organische, vor allem polare Lösungsmittel, wie Niederalkanole, z.B. Aethanol, Niederalkansäureamide, z.B. N,N-Dimethylformamid oder N-Methylpyrrolidon, Diniederalkylsulfoxyde, wie Dimethylsulfoxyd, bei der Verwendung quaternärer Ammoniumbasen als Kondensationsmittel ferner wasserhaltige Zweiphasensysteme, wie Methylenchlorid-Wasser, Benzol-Wasser oder Amylalkohol-Wasser.

Wasserbindende Mittel sind beispielsweise Anhydride oder innere Anhydride, wie Dicyclohexylcarbodiimid oder Phosphorpentoxid.

Die Ausgangsstoffe der Formel II werden vorteilhaft unter den Reaktionsbedingungen in situ hergestellt, beispielsweise, indem man eine Verbindung der Formel

$$R_A' - Ph - R_2 \qquad \text{(IIa)},$$

worin $R_A'$ einen in 1- und 5-Stellung einen Rest X und in 1,2-Stellung eine zusätzliche Doppelbindung oder in 1,2- und 1,5-Stellung je eine zusätzliche Doppelbindung aufweisenden, gegebenenfalls einfach ungesättigten n-Pentylrest bedeutet, oder ein Salz davon, mit gegebenenfalls im Phenylteil substituiertem Benzylamin oder einem Salz davon umsetzt.

Die neuen Verbindungen können ferner hergestellt werden, indem man eine Verbindung der Formel

$$R_B - Ph - R_2 \quad (III),$$

worin $R_B$ einen 1-unsubstituierten, gegebenenfalls einfach ungesättigten Piperidylrest bedeutet, oder ein Salz davon durch Umsetzung mit einem den gegebenenfalls substituierten Benzylrest einführenden Mittel in 1-Stellung substituiert und gewünschtenfalls ein erhaltenes Stereoisomerengemisch in die Komponenten auftrennt, die erhaltene Verbindung in eine andere Verbindung der Formel I überführt und/oder eine erhaltene freie Verbindung in ein Salz oder ein erhaltenes Salz in die freie Verbindung oder in ein anderes Salz umwandelt.

Den gegebenenfalls substituierten Benzylrest einführende Mittel sind beispielsweise reaktionsfähige Ester, wie Mineralsäureester, z.B. Halogen-, vorzugsweise Chlor-, Brom- oder Jodwasserstoffsäureester, oder organische Sulfonsäureester, z.B. Methan-, Aethan-, Aethen-, Benzol-, p-Toluol- oder p-Brombenzolsulfonsäureester entsprechender Benzylalkohole oder unter reduzierenden Bedingungen entsprechende Benzaldehyde.

Die Einführung des gegebenenfalls substituierten Benzylrestes erfolgt durch Umsetzung mit den obengenannten Mitteln in üblicher Weise, erforderlichenfalls in einem inerten Lösungsmittel, in Gegenwart eines Kondensationsmittels und/oder bei erhöhter oder erniedrigter Temperatur, z.B. bei etwa 0° bis etwa 150°C.

Bei der Umsetzung mit reaktionsfähigen Benzylestern arbeitet man in einem gegenüber den Reaktionspartnern inerten Lösungsmittel, erforderlichenfalls in Gegenwart eines basischen Kondensationsmittel, bei erhöhter oder erniedrigter Temperatur, z.B. bei etwa 0° bis 150°C,

und/oder unter Inertgas, wie Stickstoff. Basische Kondensationsmittel sind beispielsweise anorganische Basen, wie Alkalimetall- oder Erdalkalimetallhydroxide oder -carbonate, z.B. Natriumhydroxid, Kaliumhydroxid, Calciumhydroxid, Kaliumcarbonat oder Calciumcarbonat, oder tertiäre organische Stickstoffbasen, wie Pyridin oder Triäthylamin. Geeignete Lösungsmittel sind beispielsweise Ketone, wie Diniederalkylketone, z.B. Aceton, Alkohole, wie Niederalkanole, z.B. Methanol, Aethanol oder Amylalkohol, gegebenenfalls halogenierte Kohlenwasserstoffe, wie Benzol, Toluol oder Methylenchlorid, oder N,N-disubstituierte Niederalkansäureamide, wie Dimethylformamid oder N-Methylpyrrolidon.

Bei der Umsetzung mit Benzaldehyden unter reduzierenden Bedingungen verwendet man als Reduktionsmittel für Aminoalkylierungen übliche Mittel, wie Dileichtmetallhydride, z.B. Natriumborhydrid oder vorzugsweise Natriumcyanoborhydrid, katalytisch aktivierten Wasserstoff, wie Wasserstoff in Gegenwart eines Hydrierungskatalysators, wie einer Palladium-, Platin- oder Nickelkatalysators, z.B. von Platin, gegebenenfalls auf Kohle, Platinoxid, Palladium auf Kohle oder Raney-Nickel, ferner Ameisensäure oder deren Salze. Die Umsetzung in Gegenwart der genannten Reduktionsmittel erfolgt in der für diese jeweils üblichen Weise, vorteilhaft in einem gegenüber den Reaktionsteilnehmern inerten Lösungsmittel, unter neutralen oder erforderlichenfalls, schwach sauren Bedingungen, bei katalytischen Hydrierungen erforderlichenfalls unter erhöhtem Druck, z.B. bei bis etwa 10 bar. Als inerte Lösungsmittel kommen vorzugsweise Alkohole, wie Niederalkanole, z.B. Methanol oder Aethanol, gegebenenfalls im Gemisch mit Wasser in Betracht.

Bei der Verwendung von Dileichtmetallhydriden arbeitet man vorteilhaft unter neutralen oder im Falle

von Natriumcyanoborhydrid schwach sauren Bedingungen, z.B. etwa bei pH 3 bis 6, während man katalytische Hydrierungen vorteilhaft in Gegenwart einer Mineralsäure, z.B. von Salzsäure durchführt bzw. die Reaktionskomponente der Formel III in Form eines entsprechenden Säureadditionssalzes, z.B. als Hydrochlorid umsetzt.

Die Ausgangsstoffe der Formel III können nach an sich bekannten Methoden hergestellt werden. Verbindungen der Formel III, in denen $R_2$ in α-Stellung durch Oxo substituiertes Niederalkyl bedeutet, werden z.B. erhalten, indem man eine Verbindung der Formel

$$R_B' - Ph - H \quad \text{(IIIa)},$$

worin $R_B'$ einen am Stickstoffatom geschützten, wie acylierten, z.B. niederalkanoylierten, gegebenenfalls einfach ungesättigten Piperidylrest bedeutet, in Gegenwart einer Lewissäure, z.B. von Aluminiumtrichlorid, mit einem reaktionsfähigen Niederalkansäurederivat, z.B. einem Niederalkancarbonsäureanhydrid oder -chlorid, umsetzt und die Schutzgruppe in üblicher Weise, z.B. durch säurekatalysierte Hydrolyse,abspaltet.

Verbindungen der Formel III, in denen $R_2$ Formyl bedeutet, können vorteilhafter nach üblichen Formylierungsmethoden erhalten werden, z.B. durch Umsetzung einer Verbindung der Formel

$$R_B - Ph - H \quad \text{(IIIb)}$$

mit N,N-disubstituierten Formamiden, wie N-Methyl-formanilid oder Dimethylformamid, in Gegenwart von Phosphoroxychlorid oder mit Dimethylformamid-methosulfat. Verbindungen der Formel III, worin $R_2$ durch Hydroxy substituiertes Niederalkyl ist, können z.B. ausgehend von entsprechenden Verbindungen der Formel III, worin $R_2$ durch Oxo substituiertes Niederalkyl ist, durch Reduktion, z.B. mit Natriumborhydrid in einem Niederalkanol, erhalten werden. Verbindungen der Formel III, worin $R_2$ terminal-hydroxy-substituiertes Niederalkyl ist, können analog durch Reduktion von Estern, wie Niederalkylester, entsprechender ω-Carboxyniederalkylphenylpiperidinverbindungen, z.B. mit Lithiumaluminiumhydrid in Diäthyläther oder Tetrahydrofuran, hergestellt werden. Verbindungen der Formel III, worin $R_2$ Niederalkyl ist, können beispielsweise aus als Rest $R_2$ durch Hydroxy substituiertes Niederalkyl aufweisenden Verbindungen der Formel III durch Reduktion von $R_2$ zu Niederalkyl erhalten werden, vorzugsweise durch Einwirkung von Wasserstoff in Gegenwart eines Hydrierungskatalysators, z.B. mit Palladium auf Kohle, vorteilhaft in einer organischen Säure, wie Essigsäure.

Die neuen Verbindungen kann man ferner herstellen, indem man in einer Verbindung der Formel

$$R_C - Ph - R_2 \quad (IV),$$

worin $R_C$ einen durch mindestens einen durch Wasserstoff ersetzbaren Rest substituierten und/oder mindestens eine gegebenenfalls zusätzliche Doppelbindung aufweisenden Rest $R_1$ bedeutet, oder einem Salz davon den Rest $R_C$ zu $R_1$ reduziert und gewünschtenfalls ein erhaltenes Stereoisomerengemisch in die Komponenten auftrennt, die erhaltene Verbindung in eine andere Verbindung der Formel I überführt und/oder eine erhaltene freie Verbindung in ein Salz oder ein erhaltenes Salz in die freie Verbindung oder in ein anderes Salz umwandelt.

Durch Wasserstoff ersetzbare Gruppen sind beispielsweise reduktiv durch Wasserstoff ersetzbare Gruppen, wie gegebenenfalls funktionell abgewandelte Oxo-, Hydroxy- oder Mercaptogruppen oder organische Sulfonylgruppen. Funktionell abgewandelte Oxogruppen sind beispielsweise Oxo- oder Thionogruppen, Semicarbazonogruppen oder gegebenenfalls in β-Stellung durch organische Sulfonyl, wie

Benzol-, p-Toluol-, p-Brombenzol- oder Methansulfonyl, substituierte Hydrazonogruppen.

Funktionell abgewandelte Hydroxygruppen sind beispielsweise verätherte oder veresterte Hydroxygruppen, während als funktionell abgewandelte Mercaptogruppen insbesondere verätherte Mercaptogruppen in Betracht kommen. Verätherte Hydroxygruppen sind beispielsweise Niederalkoxygruppen, wie Methoxy oder Aethoxy. Veresterte Hydroxygruppen sind beispielsweise mit einer Mineralsäure oder organischen Carbon- oder Sulfonsäure veresterte Hydroxygruppen. Organische Carbonsäuren sind z.B. gegebenenfalls substituierte Benzoesäuren, Alkan-, vor allem Niederalkancarbonsäuren, z.B. Benzoe- oder Essigsäure. Organische Sulfonsäuren sind z.B. Benzol-, p- Toluol-, p-Brombenzol-, Methan-, Aethan- oder Aethensulfonsäure. Mineralsäuren sind vorzugsweise Halogenwasserstoffsäuren, z.B. Chlor-, Brom- oder Jodwasserstoffsäure. Verätherte Mercaptogruppen sind beispielsweise niederalkylierte oder niederalkenylierte Mercaptogruppen, wie Methylthio, Aethylthio oder Aethylenthio.

Gegebenenfalls funktionell abgewandelte Oxogruppen befinden sich insbesondere in α-Stellung zum Stickstoffatom. Gegebenenfalls veresterte Hydroxygruppen und verätherte Mercaptogruppen sind insbesondere an benzylische C-Atome gebunden. Verätherte Hydroxygruppen befinden sich insbesondere am C-Atom einer C-N-Doppelbindung. Gegebenenfalls zusätzliche Doppelbindungen können sowohl exo- als auch endo-cyclisch orientiert sein.

Die reduktive Ueberführung von $R_C$ in $R_1$ erfolgt in üblicher Weise durch Umsetzung mit einem geeigneten Reduktionsmittel.

Als Reduktionsmittel kommen insbesondere in Betracht: Nascierender, beispielsweise durch Einwirkung einer Verbindung mit labilem Wasserstoff auf Metalle, z.B.

einer Protonensäure, wie einer Halogenwasserstoffsäure oder Niederalkancarbonsäure, auf Eisen oder gegebenenfalls amalgamiertes Zink, Magnesium oder Aluminium, oder von Wasser auf, vorzugsweise amalgamiertes Aluminium, Magnesium oder Natrium, z.B. auf Natriumamalgam, erzeugter oder, beispielsweise durch einen Hydrierungskatalysator, wie einen Nickel- oder Edelmetallkatalysator, z.B. durch Raney-Nickel oder gegebenenfalls in chemisch oder an einen Träger gebundener Form, z.B. als Oxyd, vorliegendes Platin, wie durch Platin auf Kohle oder durch Platinoxyd, oder durch homogene Edelmetallkatalysatoren, wie Triphenylphosphin-Platinchlorid oder Triphenylphosphin-Rhodiumchlorid, katalytisch erregter Wasserstoff, ferner niedrigwertige Uebergangsmetallverbindungen, wie Zinn-II- oder Chrom-IIsalze, z.B. Zinn-II-chlorid, oder Hydride wie Calciumhydrid oder der Borhydrid-Tetrahydrofurankomplex, oder Dileichtmetallhydride, wie Lithiumaluminiumhydrid, gegebenenfalls im Gemisch mit Aluminiumchlorid, Natrium-bis-(2-methoxyäthoxy)-aluminiumhydrid oder Natrium-tris-(2-dimethylaminoäthoxy)-aluminiumhydrid, Natriumborhydrid oder Natriumcyanoborhydrid.

Die Umsetzung kann in aus der Literatur jeweils als geeignet bekannter Weise erfolgen.

Gegebenenfalls veresterte oder verätherte, an ein benzylisches Kohlenstoffatom gebundene Hydroxygruppen, ketonische Oxogruppen und/oder mindestens eine Doppelbindung aufweisende Gruppen $R_C$ können insbesondere durch übliche Umsetzung mit, z.B. wie vorstehend angegeben, katalytisch erregtem Wasserstoff, beispielsweise mit Wasserstoff in Gegenwart von Palladium auf Kohle, erforderlichenfalls in einem inerten Lösungsmittel, wie einem Niederalkanol, einer Niederalkansäure oder einem aliphatischen Aether, z.B. in Aethanol, Essigsäure oder Dioxan, und/oder bei erhöhter Temperatur, reduziert werden.

Ketonische Oxogruppen, Sulfonylgruppen und/oder verätherte Mercaptogruppen aufweisende Reste $R_C$ können ferner durch übliche Umsetzung mit, z.B. wie vorstehend angegeben erzeugten, nascierendem Wasserstoff, beispielsweise nach der Verfahrensweise von Clemmensen, vorzugsweise mit Zink und Salzsäure, reduziert werden.

Halogen, an das C-Atom einer C-N-Doppelbindung gebundenes veräthertes Hydroxy, mindestens eine, gegebenenfalls zusätzliche Doppelbindung und/oder lactamische bzw. amidische Oxogruppen aufweisende Reste $R_C$ kann man beispielsweise durch übliche Umsetzung mit einem geeigneten Dileichtmetallhydrid, wie einem der genannten, erforderlichenfalls in einem inerten Lösungsmittel und/oder bei erhöhter Temperatur, z.B. bei Siedetemperatur, ausgehend von Halogenverbindungen beispielsweise mit Natriumborhydrid in Wasser, Alkoholen, wie Aethanol, oder Aethylenglykomomethyläther, oder Aminen, wie Pyridin, Triäthylamin, mit Natrium-bis-(2-methoxyäthoxy)-aluminiumhydrid in aromatischen oder araliphatischen Kohlenwasserstoffen, wie Benzol oder Toluol, oder mit Natrium-tris-(dimethylaminoäthoxy)-aluminiumhydrid, oder ausgehend von Lactamen oder Amiden beispielsweise mit Lithiumaluminiumhydrid in einem aliphatischen Aether, z.B. in Diäthyläther, Tetrahydrofuran oder Dioxan, erforderlichenfalls in der Siedehitze reduzieren.

Wie angegeben substituierte Hydrazonogruppen, z.B. β-(p-Toluolsulfonyl)-hydrazono, aufweisende Reste $R_C$ können insbesondere durch übliche Umsetzung mit einem Dileichtmetallhydrid, z.B. mit Natriumcyanoborhydrid in Hexamethylphosphorsäuretriamid, erforderlichenfalls bei erhöhter Temperatur, durch Wasserstoff ersetzt werden. Semicarbazono- oder unsubstituierte Hydrazonogruppen aufweisende Reste $R_C$ können beispielsweise durch übliche Umsetzung mit einer starken Base, beispielsweise nach der Verfahrensweise von Wolff-Kishner, mit einem Alkalialkoholat, z.B. mit Natriummethanolat, erforderlichenfalls unter erhöhtem Druck und/oder bei erhöhter Temperatur oder nach der Modifikation von Huang-Minlon mit einem Alkalimetallhydroxid, z.B. Kaliumhydroxid, in einem inerten, hochsiedenden Lösungsmittel, z.B. in Di- oder Triäthylenglykol oder Diäthylenglykolmonomethyläther, reduziert werden.

In einer bevorzugten Ausführungsform des vorstehenden Verfahrens geht man beispielsweise von einer Verbindung der Formel IV aus, worin der Rest $R_C$ einen mindestens eine an das Stickstoffatom gebundene Carbonylgruppe aufweisenden Rest $R_1$, beispielsweise einen in 1-Stellung durch gegebenenfalls substituiertes Benzoyl substituierten Piperidyl- oder 2-Piperidonrest oder einen in 1-Stellung durch gegebenenfalls substituiertes Benzyl substituierten 2-Piperidonrest, bedeutet aus, und reduziert die Oxogruppe(n) durch Umsetzung mit einem geeigneten Dileichtmetallhydrid, z.B. mit Lithiumaluminiumhydrid in einen Aether, z.B. in Diäthyläther oder Tetrahydrofuran, erforderlichenfalls bei erhöhter Temperatur, z.B. in der Siedehitze.

In einer anderen bevorzugten Ausführungsform geht man von einer Verbindung der Formel IV aus, worin $R_C$ einen gegebenenfalls im Phenylteil des Benzylrestes substituierten N-Benzylpyridiniumrest bedeutet, und reduziert mindestens zwei der Doppelbindungen des Pyridiniumrestes zu Einfachbindungen. Verwendet man als Reduktionsmittel Wasserstoff in Gegenwart eines Platin- oder Rhodiumkatalysators, z.B. von Platin auf Kohle, Platinoxyd, oder den Triphenylphosphin-Rhodiumchloridkomplex, oder Lithiumaluminiumhydrid in einen Aether, wie Diäthyläther oder Tetrahydrofuran, erhält man vorzugsweise Verbindungen der Formel I, in denen $R_1$ einen gesättigten Piperidyl-

rest aufweist. Demgegenüber erhält man bei Verwendung von milder wirkenden Dileichtmetallhydriden, z.B. von Natriumborhydrid in einem Niederalkanol, wie Aethanol, vorzugsweise Verbindungen der Formel I, worin $R_1$ einen 1,2,5,6-Tetrahydropyridylrest aufweist.

Ebenfalls durch Wasserstoff ersetzbar ist die gegebenenfalls in Salzform, z.B. als Alkalimetallsalz, wie Natriumsalz, Kupfersalz oder Ammoniumsalz, vorliegende Carboxygruppe. Diese befindet sich vorzugsweise an dem mit dem Rest $-PhR_2$ verbundenen C-Atom des Restes $R_C$. Der Ersatz derselben durch Wasserstoff erfolgt vorzugsweise durch Decarboxylierung, beispielsweise durch Erhitzen auf etwa 100 bis 250°C, erforderlichenfalls in einem hochsiedenen Lösungsmittel, z.B. in Aethylenglykol, Dimethylformamid, Aethylenglykolmonomethyläther oder Diphenyläther.

Die Ausgangsstoffe der Formel IV sind bekannt oder können nach an sich bekannten Methoden hergestellt werden.

Verbindungen der Formel IV, worin $R_C$ einen an dem mit einem Niederalkylphenylrest $-PhR_2$ verbundenen C-Atom durch Hydroxy substituierten, gegebenenfalls im Phenylteil substituierten 1-Benzylpiperidylrest bedeutet, können z.B. durch Umsetzung eines entsprechenden N-Benzylpiperidons mit einem durch Einwirkung eines Niederalkylhalogenbenzols mit Magnesium erhältlichen Niederalkylphenylmagnesiumhalogenids, beispielsweise der Formel $R_2$-Ph-MgBr, vorzugsweise in einem Aether, wie Diäthyläther oder Tetrahydrofuran, hergestellt werden. Analog können auch Ausgangsstoffe hergestellt werden, in denen $R_2$ Oxo oder Hydroxy aufweist, indem man von Oxo- oder Hydroxyniederalkylhalogenbenzolen ausgeht, in denen die Oxo- bzw. Hydroxygruppe geschützt ist, z.B. Oxo acetalisiert ist, und die Schutzgruppe nach Durchführung der Reaktionsfolge abspaltet.

Verbindungen der Formel IV, worin $R_C$ einen durch verestertes oder veräthertes Hydroxy substituierten, gegebenenfalls im Phenylteil substituierten 1-Benzylpiperidylrest bedeutet, können beispielsweise aus dem entsprechenden, z.B. wie vorstehend erhältlichen Hydroxyverbindungen erhalten werden, indem man die Hydroxygruppen in üblicher Weise verestert oder veräthert, z.B. mit einem Halogenierungsmittel, wie Thionylchlorid oder Phosphortribromid, in Halogen oder mit einem Niederalkancarbonsäureanhydrid, wie Acetylchlorid, in Niederalkanoyloxy oder durch Umsetzung mit einem Niederalkanolat, wie Natriumäthanolat, in Niederalkoxy überführt.

Verbindungen der Formel IV, in denen der Rest $R_C$ eine verätherte Mercaptogruppe aufweist, können beispielsweise erhalten werden, indem man eine entsprechende, z.B. wie vorstehend angegeben erhältliche, Halogenverbindung in üblicher Weise mit einem Alkalimetallniederalkylthiolat, umsetzt.

Verbindungen der Formel IV, in denen der Rest $R_C$ eine Sulfonylgruppe aufweist, sind beispielsweise zugänglich durch übliche Oxidation entsprechender Thioäther, vorzugsweise mit einer organischen Persäure, wie m-Chlorperbenzoesäure. An dem mit dem Rest $-Ph-R_2$ verbundenen C-Atom durch Sulfonyl substituierte Verbindungen der Formel IV können ferner erhalten werden, indem man ein die Partialstruktur $R_2-Ph-CH_2-SO_2-$ aufweisendes Benzylsulfon mit einem entsprechenden 1,5-Dihalogen-, wie 1,5-Dibrom-N-benzyl-pentan bzw. -penten in üblicher Weise kondensiert.

In analoger Weise können auch Verbindungen der Formel IV, in denen der Rest $R_C$ an dem mit dem Rest $-PhR_2$ verbundenen C-Atom eine Carboxygruppe aufweist, erhalten werden, indem man in üblicher Weise einen die Partialstruktur $R_2-Ph-CH_2-COO-$ aufweisenden Phenylessigsäureester mit einem entsprechenden 1,5-Dihalogen-N-benzyl-pentan bzw.

-penten kondensiert.

Verbindungen der Formel IV, worin $R_C$ einen gegebenenfalls im Phenylteil substituierten 1-Benzyl-2-piperidonrest bedeutet, können beispielsweise hergestellt werden, indem man eine entsprechende 5-Benzylamino-($R_2$-phenyl)-valeriansäure oder ein reaktives Derivat, wie einen Ester oder ein Anhydrid davon, in üblicher Weise cyclisiert. In analoger Weise kann man auch Verbindungen der Formel IV herstellen, in denen $R_C$ einen gegebenenfalls substituierten 1-Benzyl-2,6-dioxopiperidylrest bedeutet. Dabei geht man von einer entsprechenden $R_2$-Phenylglutarsäure oder einem reaktiven Derivat, z.B. einem Diester oder Anhydrid, davon aus und setzt in üblicher Weise mit einem entsprechenden Benzylamin um.

Verbindungen der Formel IV, in denen $R_C$ funktionell abgewandeltes Oxo aufweist, können beispielsweise erhalten werden, indem man in entsprechenden Oxoverbindungen die Oxogruppe(n) in üblicher Weise funktionell abwandelt, z.B. durch Umsetzung mit Semicarbazid oder gegebenenfalls substituiertem Hydrazin in Semicarbazono bzw. Hydrozono überführt. Analog kann man Oxoverbindungen der Formel IV durch Alkylierung, z.B. mit Triniederalkyloxoniumsalzen, in die entsprechenden Enol- oder Lactimäther überführen. Unsubstituierte Hydrazonoverbindungen der Formel IV werden dabei vorteilhaft in situ hergestellt, indem man die Umsetzung mit Hydrazin bei erhöhter Temperatur, z.B. bei etwa 100 bis 250°C, erforderlichenfalls in einem hochsiedenden Lösungsmittel, wie Di- oder Triäthylenglykol oder Diäthylenglykolmonomethyläther, und in Gegenwart einer Base, z.B. eines Metallalkoholates, wie Natriummethanolat, oder eines Alkalimetallhydroxides, z.B. von Kaliumhydroxid, vornimmt.

Verbindungen der Formel IV, in denen der Rest

$R_C$ in 1-Stellung einen gegebenenfalls substituierten Benzoylrest aufweist, können beispielsweise hergestellt werden, indem man eine Verbindung der Formel $R'_C$-Ph-$R_2$ (IVa), in denen $R'_C$ in 1-Stellung unsubstituiert ist, vorzugsweise eine Verbindung der Formel $R_B$-Ph-$R_2$ (II), worin $R_B$ einen in 1-Stellung unsubstituierten, gegebenenfalls einfach ungesättigten Piperidylrest bedeutet, in üblicher Weise mit einem Benzoylierungsmittel, z.B. mit Benzoylchlorid, umsetzt, erforderlichenfalls in Gegenwart eines basischen Kondensationsmittels, z.B. von Natronlauge, oder einer organischen Stickstoffbase, z.B. von Pyridin oder Triäthylamin. In analoger Weise können auch Verbindungen der Formel IV erhalten werden, worin $R_C$ einen gegebenenfalls substituierten N-Benzylpyridinrest bedeutet, indem man ein entsprechendes $R_2$-Phenylpyridin mit einem reaktiven Derivat, z.B. dem Bromid, eines entsprechenden Benzylalkohols quaternisiert.

Die neuen Verbindungen, in denen $R_1$ einen in 1-Stellung durch gegebenenfalls substituiertes Benzyl substituierten einfach ungesättigten Piperidylrest bedeutet, können ferner hergestellt werden, indem man in einer entsprechender Verbindung der Formel

$$R_D - Ph - R_2 \quad (V) ,$$

worin $R_D$ einen in 1-Stellung durch gegebenenfalls substituiertes Benzyl und durch einen der α,β-Eliminierung zugänglichen Rest substituierten Piperidylrest bedeutet, oder einem Salz davon diesen zusammen mit einem β-ständigen Wasserstoffatom eliminiert und gewünschtenfalls ein erhaltenes Stereoisomerengemisch in die Komponenten auftrennt, die erhaltene Verbindung in eine andere Verbindung der Formel I überführt und/oder eine erhaltene freie Verbindung in ein Salz oder ein erhaltenes Salz in die freie Verbindung oder in ein anderes Salz umwandelt.

Zusammen mit einem β-ständigen Wasserstoffatom abspaltbare Reste sind beispielsweise gegebenenfalls veresterte Hydroxygruppen, gegebenenfalls verätherte oder veresterte Mercaptogruppen, Sulfoniumgruppen, Sulfinylgruppen, Sulfonylgruppen und Ammoniumgruppen. Verestertes Hydroxy oder Mercapto ist beispielsweise mit einer Mineralsäure, wie einer Halogenwasserstoffsäure, z.B. mit Brom- oder Chlorwasserstoff, verestertes Hydroxy oder mit einer organischen Säure, wie einer Niederalkancarbonsäure, z.B. Essigsäure, oder mit Xanthogensäure, verestertes Hydroxy oder Mercapto. Veräthertes Mercapto ist beispielsweise Niederalkylthio, wie Methyltho oder Aethylthio, oder gegebenenfalls substituiertes Phenylthio, z.B. Phenyl-, p-Methylphenyl- oder p-Bromphenylthio. Sulfinyl- und Sulfonylgruppen sind beispielsweise von den vorstehend genannten verätherten Mercaptogruppen abgeleitete Sulfinyl- bzw. Sulfonylgruppen, z.B. Methan-, Aethan-, Benzol-, p-Toluolsulfinyl bzw. -sulfonyl. Sulfoniumgruppen sind beispielsweise Diniederalkylsulfoniumgruppen, wie Dimethylsulfonium. Ammoniumgruppen sind beispielsweise Diniederalkyl- oder Triniederalkylammoniumgruppen, wie Diäthylammonium oder Triäthylammonium, oder Diniederalkylaminoxidgruppierungen, wie Diäthylaminoxidgruppen.

Die Eliminierung erfolgt in üblicher Weise, erforderlichenfalls in Gegenwart katalytischer und/oder Abspaltungsprodukte bindende Mittel, bei erhöhter Temperatur, z.B. bei etwa 50 bis 200°C, unter, z.B. destillativer insbesondere azeotrop-destillativer Entfernung von Reaktionsprodukten, und/oder unter Inertgas wie Stickstoff, vorteilhaft in einem inerten Lösungsmittel. Katalytische Mittel sind beispielsweise die Eliminierung von Wasser, Schwefelwasserstoff, organischen Säuren, Ammoniak und sekundärer Amine erleichternde Mittel, wie Protonensäuren, z.B. Mineralsäuren, vorzugsweise Chlorwasserstoffsäure,

Schwefelsäure oder Alkalimetallhydrogensulfate, Phosphorsäure, p-Toluolsulfonsäure oder sauren Ionenaustauscher, oder die Eliminierung von Mineralsäuren erleichtende schwach nukleophile Basen, wie Alkalimetallhydroxide, z.B. Kaliumhydroxid. Abspaltungsprodukte bindende Mittel sind beispielsweise wasserbindende Mittel, wie Dicyclohexylcarbodiimid, oder Schwefel-, Wasserstoff- oder Mercaptanbindende Mittel, wie Schwermetallsalze, z.B. Kupfer, Zink oder Silbersalze, ferner ammoniak- bzw. aminbindende Mittel wie Mineralsäuren.

Die Ausgangsstoffe der Formel V sind bekannt oder können in an sich bekannter Weise hergestellt werden.

Verbindungen der Formel V, worin $R_D$ an dem mit dem Rest $-PhR_2$ verbundenen C-Atom Hydroxy oder Mercapto aufweist, können beispielsweise erhalten werden, indem man ein entsprechendes 1-Benzyl-piperidon bzw. 1-Benzylpiperidin-thion mit einem gegebenenfalls an einer Oxo- oder Hydroxygruppe intermediär geschützten $R_2$-Phenylmagnesiumhalogenid, z.B. der Formel $R_2$-Ph-MgBr, umsetzt.

Die neuen Verbindungen können ferner hergestellt werden, indem man in einer Verbindung der Formel

$$R_1 - Ph - R_E \quad (VI) ,$$

worin $R_E$ eine in einen Rest $R_2$ überführbare Gruppe bedeutet, oder in einem Salz davon $R_E$ in $R_2$ überführt und gewünschtenfalls ein erhaltenes Stereoisomerengemisch in die Komponenten auftrennt, die erhaltene Verbindung in eine andere Verbindung der Formel I überführt und/oder eine erhaltene freie Verbindung in ein Salz oder ein erhaltenes Salz in die freie Verbindung oder in ein anderes Salz umwandelt.

In Reste $R_2$ überführbare Gruppen sind beispielsweise von $R_2$ verschiedene und reduktiv und/oder solvoly-

tisch in $R_2$ überführbare Gruppen, beispielsweise ungesättigte und/oder durch funktionell abgewandelte Oxo und/oder funktionell abgewandeltes Hydroxy substituierte niedere aliphatische Reste.

Funktionell abgewandelte Oxogruppen sind beispielsweise Thioxo, gegebenenfalls substituierte Iminogruppen oder acetalisierte bzw. thioacetalisierte Oxogruppen, ferner hydratisierte Oxogruppen und Ester derselben.

Als Substitutenten von Imino kommen beispielsweise gegebenenfalls substituiertes Phenyl, Niederalkyl oder ferner Hydroxy oder durch 2-Sulfonylniederalkyl, wie 2-Benzolsulfonylniederalkyl oder 2-(p-Toluolsulfonyl)-niederalkyl, substituiertes Amino in Betracht. Acetalisierte Oxogruppen sind beispielsweise mit einem Niederalkanol, wie Methanol oder Aethanol, oder einem Niederalkandiol, wie Aethylen- oder Propylenglykol, acetalisierte Oxogruppen. Analog sind unter thioacetalisieren Oxogruppen beispielsweise mit einem Niederalkanthiol, wie Methyl- oder Aethylmercaptan, oder einem Niederalkandithiol,wie mit 1,2-Dimercaptoäthan oder 1,3-Dimercaptopropan, thioacetalisierte Oxogruppen zu verstehen. Ester hydratisierter Oxogruppen sind beispielsweise deren Ester mit einer Mineralsäure, wie einer Halogenwasserstoffsäure, z.B. mit Chlorwasserstoffsäure.

Funktionell abgewandelte Hydroxygruppen sind beispielsweise verätherte oder veresterte Hydroxygruppen. Veräthertes Hydroxy ist beispielsweise Niederalkoxy, wie Methoxy oder Aethoxy, oder gegebenenfalls substituiertes Phenoxy. Verestertes Hydroxy ist beispielsweise mit einer Mineralsäure, wie einer Halogenwasserstoffsäure, verestertes Hydroxy, z.B. Chlor oder Brom, mit einer organischen Sulfonsäure, wie einer Niederalkan- oder gegebenenfalls substituierten Benzolsulfonsäure verestertes Hydroxy, z.B. Methan-, Aethan-, p-Toluol-, p-Brombenzol- oder Mesitylensulfonyloxy, oder mit einer Carbonsäure, wie einer Niederalkansäure oder gegebenenfalls substituierten Benzoesäure, oder gegebenenfalls partiell veresterten oder amidierten Kohlensäure verestertes Hydroxy, wie Niederalkanoyloxy, z.B. Acetoxy, Benzoyloxy, Niederalkoxy-, z.B. Methoxycarbonyloxy oder Carbamyloxy.

Als Gruppen $R_E$ kommen insbesondere in Betracht: Niederalkenyl, wie Vinyl, Allyl oder Methallyl, Niederalkinyl, wie Aethinyl oder 1-Propinyl, gegebenenfalls veresterte oder anhydridisierte Carboxy-, Carboxyniederalkyl- und Carboxyniederalkenylgruppen, wie entsprechende Niederalkoxycarbonyl, oder Halogencarbonyl aufweisende Reste veresterte oder verätherte Hydroxyniederalk(en)ylreste, wie Niederalkanoyloxy- oder gegebenenfalls Benzoyloxycarbonylreste, z.B. α-Acetoxy- oder Benzoyloxyniederalkylreste, α- oder ω-Halogenniederalkylreste, ω-Sulfonyloxy-, z.B. ω-(p-Toluolsulfonyloxy)-niederalkylreste, oder Niederalkyläther von Niederalkyl-enolen, ferner Thioxoniederalkylreste oder geminale Diniederalkoxy- oder Niederalkylendioxy-niederalkylreste.

Die reduktive Ueberführung in $R_E$ erfolgt nach an sich bekannten Methoden, beispielswiese durch katalytische Hydrierung oder Umsetzung mit Leichtmetall- oder Dileichtmetallhydriden. So kann man Niederalkenyl, Niederalkinyl beispielsweise durch Einwirkung von katalytisch aktiviertem Wasserstoff, wie von Wasserstoff in Gegenwart eines Hydrierungskatalysators, vorzugsweise eines Nickel-, Platin oder Rhodiumkatalysators, z.B. von Raney-Nickel, Platin oder Platinoxid auf Kohle oder eines Platinchlorid- oder Rhodiumchlorid-triphenylphosphinkomplex, zu Niederalkyl reduzieren. Dabei arbeitet man in üblicher Weise vorteilhaft in einem inerten Lösungsmittel, wie einem Niederalkanol, z.B. Methanol, oder einer Niederalkancarbonsäure, z.B. Essigsäure, erforderlichenfalls bei erhöhtem Druck,

z.B. bei bis etwa 25 bar, vorzugsweise in einem geschlossenen Gefäss. Analog kann man auch Halogencarbonyl oder Halogencarbonylniederalk(en)yl zu Hydroxyniederalkyl reduzieren.

Hydrazononiederalkyl $R_E$ kann ferner in üblicher Weise durch Basebehandlung, z.B. nach der Verfahrensweise von Wolff-Kishner, mit einem Alkalimetallalkoholat, z.B. mit Natriummethanolat, erforderlichenfalls unter erhöhtem Druck bei etwa 100 bis 250°C, oder nach Huang-Minlon mit einem Alkalimetallhydroxid, z.B. von Kaliumhydroxid, in einem inerten, hochsiedenden Lösungsmittel, z.B. Di- oder Triäthylenglykol, zu Niederalkyl $R_2$ reduziert werden.

Ferner kann man beispielsweise gegebenenfalls verätherte oder anhydridisierte Carboxy- oder Carboxyniederalkylgruppen durch Umsetzung mit Leichtmetall- oder Dileichtmetallhydriden zu Oxo- oder Hydroxyniederalkyl reduzieren. Für die Reduktion zu Oxoniederalkyl verwendet man vorzugsweise Niederalkyllithiumaluminiumhydride, wie Diisobutyl- oder Triisobutyllithiumaluminiumhydrid, wohingegen man für die Reduktion zu Hydroxyniederalkyl vor allem Lithiumaluminiumhydrid in einem Aether, wie Diäthyläther oder Tetrahydrofuran verwendet. Bei den genannten Reaktionen arbeitet man vorteilhaft unter Inertgas, z.B. unter Stickstoff oder Argon, erforderlichenfalls unter Kühlen oder Erwärmen, z.B. bei etwa -20 bis +80°C. In analoger Weise kann man auch primäres Halogen bzw. Sulfonyloxy aufweisende Halogen- bzw. Sulfonyloxyniederalkylgruppen zu Niederalkyl und mit einer Carbonsäure oder der partiell veresterten oder amidierten Kohlensäure veresterte Hydroxyniederalkylgruppen zu Hydroxyniederalkyl reduzieren. Analog kann man auch wie angegeben substituiertes Hydrazononiederalkyl zu Niederalkyl reduzieren,

beispielsweise mittels Natriumborhydrid in Hexaphosphorsäuretriamid.

Ebenfalls als Reduktion aufzufassen ist die reduktive Niederalkylierung von gegebenenfalls veresterten oder anhydridisierten Carboxy- oder Carboxyniederalkylgruppen $R_E$ mit Niederalkylmetallverbindungen zu Oxo- oder Hydroxyniederalkylgruppen $R_2$. Niederalkylmetallverbindungen sind beispielsweise solche, die als Metallradikal eine Gruppe der Formel $-M^I$, $-M^{II}$-Hal oder $M^{II}/2$ aufweisen, worin $M^I$ ein Metallatom der Gruppe I, $M^{II}$ ein Metallatom der Gruppe II des Periodischen Systems der Elemente und Hal Halogen, wie Chlor, Brom oder Jod bedeutet. Bevorzugte Metallradicale der genannten Art sind solche der Formeln -Li, -MgHal- und -Cd/2.

Gegebenenfalls funktionell abgewandelte Carboxygruppen sind beispielsweise in Salzform, wie in einer Alkalimetallsalzform, z.B. als Natriumsalz, vorliegende oder, vorzugsweise mit einer Halogenwasserstoffsäure, wie Fluor-, Chlor-, Brom- oder Jodwasserstoffsäure anhydridisierte Carboxygruppen, veresterte Carboxygruppen, wie Niederalkoxycarbonyl oder gegebenenfalls substituiertes Benzoyloxycarbonyl, oder disubstituierte Carbamylgruppen, wie N,N-Diniederalkylcarbamyl oder 1-Imidazolinylcarbonyl. Die Umsetzung von gegebenenfalls funktionell abgewandelten Carbonsäuren der Formel VI mit den genannten Niederalkylmetallverbindungen erfolgt in üblicher Weise, vorteilhaft in einem inerten Lösungsmittel, wie einem Aether, z.B. in Diäthyläther oder Tetrahydrofuran, einem Kohlenwasserstoff, z.B. Benzol, oder Gemischen derselben, erforderlichenfalls unter Kühlen oder gelindem Erwärmen, z.B. bei etwa -80 bis etwa 100°C, z.B. bei Siedetemperatur, und/oder unter Inertgas, z.B. unter Stickstoff. Dabei werden in Abhängigkeit von den jeweils verwendeten Ausgangsstoffen und den Reaktionsbedingungen entweder

Ketone ($R_2$ = Oxoniederalkyl) oder tertiäre Alkohole ($R_2$ = Hydroxyniederalkyl) der Formel I erhalten. So werden bei Verwendung reaktiver Niederalkylmetallverbindungen, z.B. von Niederalkylmagnesiumhalogeniden oder Lithiumniederalkylen, und bei normaler oder erhöhter Temperatur, z.B. bei etwa 0°C bis etwa 100°C, vorwiegend Verbindungen der Formel I, worin $R_2$ Hydroxyniederalkyl ist und bei erniedrigter Temperatur, z.B. bei etwa -80 bis 0°C, oder bei Verwendung wenig reaktiver Niederalkylmetallverbindungen, z.B. von Niederalkylcadmiumverbindungen, unter normalen Temperaturbedingungen vorzugsweise Verbindungen der Formel I erhalten, worin $R_2$ Oxoniederalkyl ist. In analoger Weise können auch Verbindungen der Formel I, in denen $R_2$ Niederalkyl ist, erhalten werden, indem man eine Verbindung der Formel VI, worin $R_E$ Halogen- oder Sulfonyloxyniederalkyl ist, mit einer der genannten Niederalkylmetallverbindungen, insbesondere einem Lithiumniederalkyl, umsetzt.

Durch Solvolyse kann man beispielsweise mindestens eine funktionell abgewandelte Oxo- und/oder Hydroxygruppe aufweisende Reste $R_E$ in eine Oxo- oder Hydroxygruppe aufeweisende Reste $R_2$ überführen. Beispielsweise kann man Verbindungen der Formel VI, worin $R_E$ Thioxo, gegebenenfalls substituiertes Imino, geminale oder an eine Doppelbindung gebundene verätherte Hydroxygruppen aufweist, zu Verbindungen der Formel I, worin $R_2$ Oxoniederalkyl bedeutet, oder Verbindungen der Formel VI, worin $R_E$ verestertes Hydroxy aufweist, zu Verbindungen der Formel I, worin $R_2$ Hydroxyniederalkyl ist, hydrolysieren. Die Hydrolyse erfolgt in üblicher Weise, beispielsweise in Gegenwart eines basischen oder sauren Hydrolysemittels, erforderlichenfalls in Gegenwart eines Lösungsmittels, und/oder bei erhöhter Temperatur, z.B. bei etwa 20 bis 150°C.

Basische Kondensationsmittel sind beispielsweise Alkalimetall-, Ammonium- oder Erdalkalimetallhydroxide oder entsprechende Carbonate, z.B. Natrium-, Kalium- oder Calciumhydroxid, Ammoniak oder Kalium- oder Natriumcarbonat. Saure Hydrolysemittel sind beispielsweise Protonensäuren, wie Mineralsäuren, z.B. Salz- oder Schwefelsäure, Sulfonsäuren, z.B. p-Toluolsulfonsäure, oder Carbonsäuren, wie Essigsäure. Inerte Lösungsmittel sind beispielsweise mit Wasser mischbare Lösungsmittel, wie Niederalkanole, z.B. Methanol oder Aethanol, Ketone, z.B. Aceton, Amide, z.B. Dimethylformamid oder N-Methylpyrrolidon, oder Sulfoxide, z.B. Dimethylsulfoxid. Bei der Hydrolyse von Thioxoniederalkyl arbeitet man ferner erforderlichenfalls in Gegenwart einer Schwermetallverbindung, z.B. von Kupferoxid, oder eines Oxidationsmittels, z.B. von Wasserstoffperoxid oder organischen Persäuren.

Mit einer Carbonsäure veresterte Hydroxyniederalkylreste $R_E$ können ferner durch Umsetzung mit einem niedermolekularen Alkohol, wie einem Niederalkanol, vor allem Methanol, in Gegenwart einer Mineralsäure, z.B. von Chlorwasserstoffsäure, oder eines Alkalimetallalkoholates, wie Natriummethanolat alkoholytisch oder durch Umsetzung mit Ammoniak oder einem organischen Amin, aminolytisch in Hydroxyniederalkyl überführt werden.

Die Ausgangsstoffe der Formel VI können nach an sich bekannten Methoden hergestellt werden.

So kann man Verbindungen der Formel VI, in denen $R_E$ eine funktionell abgewandelte Oxo- und/oder Hydroxygruppe aufweist, beispielsweise herstellen, indem man in einer Verbindung der Formel $R_B$-Ph-$R_2$ (III), worin $R_B$ einen 1-unsubstituierten, gegebenenfalls einfach ungesättigten Piperidylrest bedeutet, die Gruppe $R_2$ durch Ueberführung

von Oxo mit Phosphorpentasulfid oder Aluminiumtrisulfid in Thioxo, durch Umsetzung mit Ammoniak oder einem organischen Amin, Hydroxylamin oder gegebenenfalls substituiertem Hydrazin in eine gegebenenfalls substituierte Iminogruppe, mit einem Orthoester in acetalisiertes bzw. mit einem Niederalkanthiol in thioacetalisiertes Oxo aufweisendes $R_E$ und/oder von Hydroxy durch Veresterung in verestertes Hydroxy aufweisendes $R_E$ überführt und anschliessend, z.B. durch Umsetzung mit einem gegebenenfalls substituierten Benzylbromid den Rest $R_B$ in den gewünschten Rest $R_1$ überführt.

Verbindungen der Formel VI, worin $R_E$ eine acetalisierte bzw. thioacetalisierte oder in Enolätherform vorliegende Oxogruppe aufweist, kann man beispielsweise ferner herstellen, indem man ein entsprechendes Halogenphenyloxoniederalkan, z.B. der Formel Halogen-Ph-$R_2$ (VIa) durch Umsetzung mit einem Orthoester acetalisiert bzw. durch Umsetzung mit einem Mercaptan oder Dimercaptan thioacetalisiert oder in üblicher Weise enolveräthert, das Produkt, z.B. der Formel Halogen-Ph-$R_E$ (VIb), mit Magnesium in die Grignard-Verbindung, z.B. der Formel Halogen-Mg-Ph-$R_E$ (VIc), überführt, diese mit einem gegebenenfalls substituierten 1-Benzal-halogen-piperidin umsetzt und basisch aufarbeitet. Aus diesen können durch Umsetzung mit Schwefelwasserstoff entsprechende Verbindungen der Formel VI erhalten werden, in denen $R_E$ Thioxo aufweist.

Verbindungen der Formel VI, in denen $R_E$ Carboxy bzw. Carboxyniederalkyl bedeutet, kann man ferner herstellen, indem man eine Halogenbenzoesäure bzw. Halogenphenyl-niederalkansäure, in das Säurechlorid überführt und mit Aminoisobutanol bzw. 2,2-Dimethylaziridin in das entsprechende 2-(Halogenphenyl)- bzw. 2-(Halogenphenylniederalkyl)-4,4- bzw. -5,5-dimethyl-oxazolyl-(2) umsetzt oder ein entsprechendes Nitril unter Säurekatalyse mittels 4-Amino-2-methyl-pentan-2-ol oder 2-Methyl-pentan-2,4-diol in das entsprechende 2-(Halogenphenyl)- bzw. 2-(Halogenphenylniederalkyl)-4,4,6-trimethyl-5,6-dihydro-oxazinyl überführt, das Reaktionsprodukt jeweils mit Magnesium in die entsprechende Grignard-Verbindung überführt, diese mit einem entsprechenden 1-Benzylpiperidylhalogenid bzw. 1-Benzylpiperidon umsetzt, im letzterem Falle aus dem Reaktionsprodukt Wasser abspaltet, jeweils die Schutzgruppe durch Säurebehandlung entfernt und gewünschtenfalls die erhaltene Säure verestert oder in ein Anhydrid, z.B. mit Thionylchlorid in das Säurechlorid, umwandelt.

Verbindungen der Formel VI, in denen $R_E$ einen durch gegebenenfalls verestertes oder anhydridisiertes Carboxy substituierten, in 2-Stellung zu der genannten Gruppe Hydroxy aufweisenden oder in 2,3-Stellung ungesättigten Rest bedeutet, können ferner hergestellt werden, indem man ein im Piperidylteil gegebenenfalls einfach ungesättigtes 1-Acyl-phenylpiperiden mit einem Niederalkanoylhalogenid in Gegenwart von Aluminiumtrichlorid im Phenylteil niederalkanoyliert, in dem so erhältlichen Niederalkanoyl-(1-Acylpiperidyl)-phenon in üblicher Weise, z.B. durch Umsetzung mit einem Niederalkancarbonsäureester in Gegenwart eines Alkalimetallalkoholates, mit einem α-Halogenniederalkansäureester und Zink oder mit einem Niederalkancarbonsäureanhydrid in Gegenwart eines entsprechenden Alkalimetallcarboxylates, oder mittels eines Phosphorylidenessigsäureesters, wie von Triphenylphosphorylidenessigsäureäthylester, den Rest $R_E$ aufbaut, den 1-Acylrest hydrolytisch abspaltet, das Reaktionsprodukt in üblicher Weise, z.B. durch Umsetzung mit gegebenenfalls substituiertem Benzylbromid in Gegenwart von Triäthylamin, 1-benzyliert, gewünschtenfalls funktionell abgewandeltes Carboxy hydrolysiert und/oder Carboxy, z.B. mit Thionylchlorid, halogeniert.

In analoger Weise können ausgehend von 1-Acylpiperidyl-benzaldehyden bzw. 1-Acylpiperidyl-niederalkanalen, die durch Formylierung, z.B. mittels Dimethylformamid und Phosphoroxychlorid, entsprechender 1-Acylpiperidyl-benzole und gegebenenfalls Kettenverlängerung mittels Methoxymethylen-triphenyl-phosphoran, zugänglich sind, durch Umsetzung mit Niederalkanalen oder Niederalkansäureestern in Gegenwart einer starken Base, wie eines Alkalimetallniederalkanolates, Verbindungen der Formel VI erhalten werden, in denen $R_E$ eine Oxo- oder veresterte Carboxygruppe und eine dazu β-ständige Hydroxygruppe oder eine konjugierte Doppelbindung aufweist. Analog kann man in Gegenwart eines Metalls, z.B. von Zink, mit einem α-Halogenniederalkansäureester umsetzen, wobei entsprechende 2-Hydroxyniederalkansäureester erhalten werden.

Verbindungen der Formel VI, in denen $R_E$ Niederalkenyl oder Niederalkinyl bedeutet, können ferner hergestellt werden, indem man ein entsprechendes Halogenphenyl-niederalken bzw. -niederalkin in eine Metallverbindung, z.B. durch Umsetzung mit Magnesium in die entsprechende Halogenmagnesiumverbindung überführt, diese in üblicher Weise mit einem gegebenenfalls substituierten 1-Benzyl-halogenpiperidin bzw. 1-Benzylpiperidon kondensiert und im letzteren Fall Wasser abspaltet.

Verbindungen der Formel VI, in denen $R_E$ Halogen aufweist, können beispielsweise durch übliche Halogen- bzw. Halogenwasserstoffanlagerung an entsprechende, einen ungesättigten Rest $R_E$ aufweisende Verbindungen der Formel VI hergestellt werden.

Die für die Herstellung von Ausgangsstoffen der Formel VI zu verwendenden Niederalkylmetallverbindungen, werden vorteilhaft in situ hergestellt, indem man eine entsprechende Halogen-, z.B. Chlor-, Jod- oder vor allem Bromniederalken, vorzugsweise in einem Aether, z.B. in

Diäthyläther oder Tetrahydrofuran, mit Lithium oder vor allem Magnesium, umsetzt. Andere Niederalkylmetallverbindungen können aus den so erhältlichen Halogen-, vor allem Brommagnesiumverbindungen, durch Umsetzung mit einem entsprechenden Metallhalogenid, z.B. mit Cadmiumchlorid, Kupferchlorid oder Zinkchlorid, erhalten werden.

Die neuen Verbindungen kann man weiterhin herstellen, indem man auf ein Gemisch von Verbindungen der allgemeinen Formeln

$$R_o - Ph - H \quad (VII) \quad \text{und} \quad R_o' - X_1 \quad (VIII) \; ,$$

worin einer der Reste $R_o$ und $R_o'$ einen Rest $R_1$ und der andere einen Rest $R_2$ bedeutet, und $X_1$ eine gegebenenfalls reaktionsfähig veresterte Hydroxygruppe oder eine sich zu einem benachbarten Kohlenstoffatom erstreckende Bindung bedeutet, ein geeignetes saures Mittel einwirken lässt und gewünschtenfalls eine oder mehrere der genannten Zusatzoperationen durchführt.

Der Rest $X_1$ ist vor allem reaktionsfähig verestertes Hydroxy, insbesondere Halogen mit Atomnummer 17 und höher, wie Chlor. Als an Niederalkanoyl $R_1$ gebundener Rest $X_1$ kommt ferner mit der Säure der Formel $R_1$-OH verestertes Hydroxy in Betracht.

Geeignete saure Mittel sind beispielsweise Mineralsäuren, wie Fluorwasserstoffsäure oder gegebenenfalls in Anhydridform vorliegende Sauerstoffsäuren des Phosphors oder Schwefels, z.B. Phosphorsäure, Diphosphorsäure, Polyphosphorsäuren oder Phosphorpentoxid oder Schwefelsäure, oder vor allem Lewissäuren, wie Halogenide von Elementen der Hauptgruppen III, IV und V und der Nebengruppen II und VIII des Periodischen Systems der Elemente, wie des Bors, Aluminiums, Galliums, Zinns, Antimons und Eisen, z.B. neben Eisen-trichlorid, Zinkchlorid, Zinnchlorid und Antimonpentachlorid vor allem Bortrichlorid und -fluorid und Aluminiumtrichlorid und -bromid, ferner komplexe Metallsäuren, wie Tetrafluorbor- oder Hexachloroantimonsäure.

Die Umsetzung erfolgt in üblicher Weise, beispielsweise in einem inerten Lösungsmittel, wie Schwefelkohlenstoff, Nitrobenzol, Tetrachlormethan, Diäthyläther, Tetrahydrofuran oder einem Ueberschuss des Ausgangsstoffes der Formel VIII, bei normaler, mässig erhöhter oder erniedrigter Temperatur, z.B. bei etwa -30 bis etwa 100°C, vorteilhaft unter Feuchtigkeitsausschluss und/oder Schutzgas, z.B. unter Stickstoff.

In einer bevorzugten Ausführungsform des vorstehenden Verfahrens lässt man beispielsweise auf ein Gemisch von Verbindungen der Formeln VII und VIII, worin $R_o$ einen Rest $R_1$ und $R_o'-X_1$ ein Niederalkanoylhalogenid, z.B. -chlorid, oder Niederalkansäureanhydrid bedeutet, Aluminiumtrichlorid einwirken, wobei man vorzugsweise in siedendem Schwefelkohlenstoff arbeitet.

Die neuen Verbindungen kann man ferner herstellen, indem man Verbindungen der Formeln

$$R_o - Ph - X_2 \text{ (IX)} \quad \text{und} \quad R_o'' \overset{---}{=} X_3 \text{ (X)},$$

worin einer der Reste $R_o$ und $R_o''$ einen Rest $R_1$ und der andere einen Rest $R_2$ und einer der Reste $X_2$ und $X_3$ ein Metallradikal und der andere Halogen oder an einen Niederalkylrest $R_o''$ gebundenes Oxo oder an einen Niederalkanoylrest $R_o''$ gebundenes in Salzform vorliegendes veräthertes oder verestertes Hydroxy bedeutet, miteinander kondensiert und gewünschtenfalls ein erhaltenes Steroisomerengemisch in die Komponenten auftrennt, die erhaltene Verbindung in eine andere Verbindung der Formel I überführt und/oder eine erhaltene freie Verbindung in ein Salz oder ein erhaltenes Salz in die freie Verbindung oder in ein anderes Salz umwandelt.

Metallradikale sind beispielsweise Gruppender Formel $-M^{I}$, $-M^{II}$ oder $-M^{II}/2$, worin $M^{I}$ ein Metallatom der Gruppe I, $M^{II}$ ein Metallatom der Gruppe II des Periodischen Systems der Elemente und Hal Halogen, wie Chlor, Brom oder Jod bedeutet. Bevorzugte Metallradikale der genannten Art sind solche der Formeln -Li, MgHal- und -Cd/2.

Veräthertes Hydroxy ist beispielsweise Niederalkoxy. Verestertes Hydroxy ist z.B. mit einer Säure der Formel $R_o''$-OH verestertes Hydroxy. In Salzform vorliegendes Hydroxy ist beispielsweise in einer Alkalimetallsalzform, z.B. als Natriumsalz, vorliegendes Hydroxy, Halogen ist vorzugsweise Fluor, Chlor, Brom oder Jod.

Die Umsetzung von Verbindungen der Formeln IX und X erfolgt in üblicher Weise, vorteilhaft in einem inerten Lösungsmittel, wie einem Aether, z.B. in Diäthyläther oder Tetrahydrofuran, einem Kohlenwasserstoff, z.B. Benzol, oder Gemischen derselben, erforderlichenfalls unter Kühlen oder gelindem Erwärmen, z.B. bei etwa -30 bis etwa 100°C, z.B. bei Siedetemperatur, und/oder unter Inertgas, z.B. unter Stickstoff. Bevorzugte Ausführungsformen dieses Verfahrens sind insbesondere die Umsetzung von Cadmiumverbindungen (IX) mit Säurechloriden (X) in Benzol/Diäthyläther zu Verbindungen der Formel I, worin $R_2$ 1-Oxoniederalkyl ist, von Halogenmagnesiumverbindungen (IX) mit Niederalkanonen (X) zu Verbindungen der Formel I, worin $R_2$ 1-Hydroxyniederalkyl ist und von Lithium- oder Halogenmagnesiumverbindungen (X) mit Verbindungen der Formel IX, worin $R_o$ ein Rest $R_1$ und $X_1$ Halogen ist.

Die Ausgangsstoffe der Formeln IX und X sind bekannt oder können, soweit neu, nach an sich bekannten Methoden hergestellt werden.

Die als Ausgangsstoffe zu verwendenden metallorganischen Verbindungen, z.B. der Formel IX, worin $R_o$

ein Niederalkylrest $R_2$ und $X_1$ das Metallradikal darstellt, werden vorteilhaft in situ hergestellt, indem man eine entsprechende Halogen-, z.B. Chlor-, Jod- oder vor allem Bromverbindung, vorzugsweise in einem Aether, z.B. in Diäthyläther oder Tetrahydrofuran, mit Lithium oder vor allem Magnesium, umsetzt. Andere metallorganische Verbindungen können aus den so erhältlichen Halogen- vor allem Brommagnesiumverbindungen, durch Umsetzung mit einem entsprechenden Metallhalogenid, z.B. mit Cadmiumchlorid, Kupferchlorid oder Zinkchlorid, erhalten werden. Die dafür zu verwendenden Halogenverbindungen können z.B. hergestellt werden, indem man einen 1-Acylpiperidyl-benzol oder $R_2$-Benzol in üblicher Weise, z.B. mit Brom bzw. Chlor in Gegenwart von Eisen oder mit N-Chlorsuccinimid, halogeniert, ein erhaltenes 1-Acylpiperidyl-halogenbenzol hydrolysierend entacetysiert und anschliessend, z.B. mit einem entsprechenden Benzylbromid, 1-benzyliert. Gegebenenfalls substituierte 1-Benzylhalogenpiperidinverbindungen können analog durch 1-Benzylierung entsprechender 1-unsubstituierter Phenylpiperidine bzw. Phenyl-tetrahydropyridine hergestellt werden.

Erfindungsgemäss erhältliche Verbindungen der Formel I können in an sich bekannter Weise in andere Verbindungen der Formel I umgewandelt werden.

So kann man beispielsweise in Verbindungen der Formel I einen Oxo aufweisenden Rest $R_2$ zu dem entsprechenden Hydroxy aufweisenden Rest $R_2$ reduzieren. Dabei verwendet man als Reduktionsmittel beispielsweise ein Dileichtmetallhydrid, wie ein Alkalimetallborhydrid oder Alkalimetallaluminiumhydrid, z.B. Natriumborhydrid, Natriumcyanoborhydrid oder Lithiumaluminiumhydrid, vorzugsweise in einem inerten Lösungsmittel, erforderlichenfalls unter Kühlen oder Erwärmen, z.B. bei etwa 0° bis 120°C, und/oder unter Inertgas, wie Stickstoff. Vorteilhaft ist insbesondere die Reduktion mit Natriumborhydrid in einem inerten, polaren Lösungsmittel, wie einem Niederalkanol, z.B. in Methanol, Aethanol oder Butanol, oder in Dimethylformamid bzw. in, gegebenenfalls wasserhaltigen, Gemischen derselben oder mit Lithiumaluminiumhydrid in einem Aether, wie Diäthyläther, Dioxan oder Tetrahydrofuran. Die Reduktion der Oxogruppe zu Hydroxy kann ferner durch Umsetzung mit einem Keton, wie Aceton, in Gegenwart eines Aluminiumalkoholates, z.B. von Aluminiumisopropylat, insbesondere nach der Verfahrensweise von Meerwein-Ponndorf-Verley erfolgen. Oxo kann aber auch reduktiv niederalkyliert werden, beispielsweise durch Umsetzung mit einer Niederalkylalkalimetall- oder Niederalkylerdalkalimetallverbindung, wie einem Niederalkyllithium, z.B. Methyl- oder Aethyllithium, oder einem Niederalkylmagnesiumhalogenid, z.B. Methylmagnesiumbromid oder Aethylmagnesiumbromid, erfolgen, vorzugsweise in einem Aether, wie Diäthyläther.

Ferner kann man in einem Oxo oder Hydroxy aufweisenden Rest $R_2$ Oxo oder Hydroxy reduktiv durch Wasserstoff ersetzen. Oxo kann beispielsweise durch Ueberführung mit einem gegebenenfalls durch 2-Sulfonyläthyl, wie 2-Benzol-, 2-(p-Toluol)-, 2-Methan- oder 2-Mesitylensulfonyläthyl, substituierten Hydrazin in gegebenenfalls substituiertes Hydrazono und Reduktion durch Wasserstoff ersetzt werden. Als Reduktionsmittel für 2-Sulfonyläthylhydrazonogruppen verwendet man vorzugsweise ein Dileichtmetallhydrid, wie Natriumcyanoborhydrid in einem inerten, polaren Lösungsmittel, z.B. in Hexamethylphosphorsäuretriamid. Der reduktive Austausch von unsubstituiertem Hydrazono gegen Wasserstoff erfolgt vorteilhaft durch basenreduzierte Disproportionierung, beispielsweise durch Umsetzung mit einem Alkalimetallalkoholat, wie Natriummethanolat, erforderlichenfalls unter erhöhtem Druck,

z.B. bis 10 bar, oder durch Einwirkung eines Alkalimetallhydroxides, z.B. von Kaliumhydroxid, in einem inerten hochsiedenden Lösungsmittel, z.B. in Di- oder Triäthylenglykol oder Diäthylenglykolmonomethyläther, jeweils erforderlichenfalls unter Erhitzen, z.B. bei etwa 100 bis 250°C. Ketonische Oxogruppen können ferner mittels nascierenden Wasserstoffs, erzeugt z.B. durch Einwirkung einer Protonensäure, wie von Salz- oder Essigsäure, auf unedle Metalle, wie Zink, Eisen oder Aluminium, von Wasser auf, vorzugsweise amalgamiertes Aluminium, oder von Alkoholen, wie Methanol, auf Natrium, durch Wasserstoff ersetzt werden. Man kann auch niedrigwertige Metallverbindungen, wie Zinn-II- oder Chrom-II-salzen, z.B. Zinn-II-chlorid, verwenden. Hydroxy kann beispielsweise durch Einwirkung von katalytisch aktiviertem Wasserstoff, wie Wasserstoff in Gegenwart eines Hydrierungskatalysators, z.B. von Raney-Nickel oder einer Platinverbindung, wie Platinoxid oder Platin auf Kohle oder auf Bariumoxid, vorteilhaft in einem sauren Lösungsmittel, wie einer Niederalkansäure, z.B. in Essigsäure, durch Wasserstoff ersetzt werden.

In Verbindungen der Formel I kann in dem Rest $R_2$ ferner oxydativ Hydroxy oder Oxo eingeführt und/oder Hydroxy in Oxo überführt werden. Die Einführung von Hydroxy, insbesondere in 1-Stellung erfolgt beispielsweise durch Luftoxidation, oder Einführung von Halogen, insbesondere durch Umsetzung mit N-Bromsuccinimid, und anschliessende Hydrolyse. Die oxydative Einführung von Oxo, ebenfalls vorzugsweise in 1-Stellung, erfolgt beispielsweise durch Umsetzung mit Selendioxid. Im Rest $R_2$ kann ferner Hydroxy zu Oxo oxydiert werden, beispielsweise durch Umsetzung mit einem Keton, wie Aceton, in Gegenwart eines Aluminiumalkoholates, wie von Aluminiumisopropylat, vorzugsweise nach der Verfahrensweise von Oppenauer.

Weiterhin kann man Oxoniederalkylreste $R_2$ in üblicher Weise, vorzugsweise durch Umsetzung mit einer Metall- oder Phosphorverbindung eines Diniederalkyl- oder Phenylniederalkyläthers, z.B. mit Methoxymethylmagnesiumbromid oder mit Methoxyphosphoran, und anschliessende Hydrolyse des erhaltenen Enoläthers, verlängern.

Ferner kann man in Verbindungen der Formel I, worin $R_1$ einen durch gegebenenfalls substituiertes Benzyl 1-substituierten, einfach ungesättigten Piperidylrest bedeutet, die Doppelbindung desselben reduzieren, beispielsweise durch Umsetzung mit katalytisch aktiviertem Wasserstoff, z.B. mit Wasserstoff in Gegenwart eines Nickel-, Platin- oder Rhodiumkatalysators, z.B. von Raney-Nickel, Platinoxid oder dem Komplex von Platin- oder Rhodiumchlorid und Triphenylphosphin, oder mittels eines Dileichtmetallhydrides, z.B. mit Natriumborhydrid oder mit Lithiumaluminiumhydrid.

Die neuen Verbindungen können, je nach der Wahl der Ausgangsstoffe und Arbeitsweisen, in Form eines der möglichen Isomeren oder als Gemisch derselben, z.B. je nach der Anzahl der asymmetrischen Kohlenstoffatome als reine optische Isomere, wie Antipoden oder als Isomerengemische, wie Racemate, Diastereoisomerengemische oder Racematgemische, vorliegen.

Erhaltene Isomerengemische, wie Diastereomerengemische und Racematgemische können auf Grund der physikalisch-chemischen Unterschiede der Bestandteile in bekannter Weise in die reinen Isomeren, Diastereomeren oder Racemate aufgetrennt werden, beispielsweise durch Chromatographie und/oder fraktionierte Kristallisation.

Erhaltene Racemate lassen sich ferner nach bekannten Methoden in die optischen Antipoden zerlegen, beispielsweise durch Umkristallisation aus einem optisch aktiven Lösungsmittel, mit Hilfe von Mikroorganismen, oder

durch Umsetzen eines Endstoffes mit einer mit der racemischen Base Salze bildenden optisch aktiven Säure und Trennung der auf diese Weise erhaltenen Salze, z.B. auf Grund ihrer verschiedenen Löslichkeiten, in die Diastereomeren, aus denen die Antipoden durch Einwirkung geeigneter Mittel freigesetzt werden können. Vorteilhaft isoliert man den wirksameren der beiden Antipoden.

Erhaltene freie Verbindungen der Formel I können in an sich bekannter Weise in Salze überführt werden, u.A. durch Behandeln mit der entsprechenden Säure, üblicherweise in Gegenwart eines Lösungs- oder Verdünnungsmittels.

Erhaltene Salze können in an sich bekannter Weise in die freien Verbindungen umgewandelt werden, z.B. durch Behandeln mit einer Base, wie einem Alkalimetallhydroxid.

Die Verbindungen einschliesslich ihrer Salze können auch in Form ihrer Hydrate erhalten werden oder das zur Kristallisation verwendete Lösungsmittel einschliessen.

Infolge der engen Beziehung zwischen den neuen Verbindungen in freier Form und in Form ihrer Salze sind im vorausgegangenen und nachfolgend unter den freien Verbindungen oder ihren Salzen sinn- und zweckgemäss gegebenenfalls auch die entsprechenden Salze bzw. freien Verbindungen zu verstehen.

Die Erfindung betrifft auch diejenigen Ausführungsformen des Verfahrens, nach denen man von einer auf irgendeiner Stufe des Verfahrens als Zwischenprodukt erhältlichen Verbindung ausgeht und die fehlenden Schritte durchführt oder einen Ausgangsstoff in Form eines Salzes und/oder Racemates bzw. Antipoden verwendet oder insbesondere unter den Reaktionsbedingungen bildet.

Beim Verfahren der vorliegenden Erfindung werden vorzugsweise solche Ausgangsstoffe verwendet, welche zu den eingangs als besonders wertvoll geschilderten Verbindungen führen. Neue Ausgangsstoffe und Verfahren zu ihrer Herstellung bilden ebenfalls einen Gegenstand der vorliegenden Erfindung.

In diesem Zusammenhang sind insbesondere die als Ausgangsstoffe genannten Verbindungen der Formel VII, in denen $R_o$ einen Rest $R_1$ bedeutet, d.h. Verbindungen der Formel $R_1$-Ph-H (Ia), in denen $R_1$ und Ph die eingangs angegebenen Bedeutungen haben, zu erwähnen. Diese weisen die gleichen pharmakologischen Eigenschaften, in vergleichbarer Wirkungsstärke auf, wie die erfindungsgemässen Verbindungen der Formel I. Die Erfindung betrifft dementsprechend ebenfalls Verbindungen der Formel Ia und ihre pharmazeutisch verwendbaren Salze, Verfahren zu ihrer Herstellung, ihre Verwendung und diese enthaltende pharmazeutische Präparate.

Diesbezüglich betrifft die Erfindung insbesondere diejenigen Verbindungsgruppen der Formel Ia, in denen $R_1$ und Ph die eingangs für bevorzugte Verbindungsgruppen angegebenen Bedeutungen haben, namentlich das 1-(o-Chlorbenzyl)-3-phenyl-piperidin und seine pharmazeutisch verwendbaren Salze, wie dessen Hydrochlorid.

Die Verbindungen der Formel Ia können in an sich bekannter Weise hergestellt werden, beispielsweise nach dem vorstehend für die Herstellung von Verbindungen der Formel I angegebenen Verfahren, indem man von anstelle des Restes $R_2$ jeweils Wasserstoff aufweisenden Verbindungen der Formeln II, IIa, III, IV, V, VII und VIII bzw. IX und X, wobei $R_o$ Wasserstoff und $R_o'$ bzw. $R_o''$ einen Rest $R_1$ bedeutet, ausgeht.

Die Verbindungen der Formel I und ihre pharmazeutisch verwendbaren Salze weisen wertvolle pharmakologische Eigenschaften auf. So besitzen sie beispielsweise ausgeprägte antithrombotische Aktivität. Diese kann beispielsweise am Meerschweinchen anhand der Hemmung der Thrombocytopenie nach Induktion durch ADP im Dosisbereich von etwa 100 bis 300 mg/kg p.o. ebenfalls anhand der Arthus-Reaktion, siehe dazu Brit. J. Pharmacology <u>57</u>, 441P (1976), im Dosisbereich von etwa 30 bis 300 mg/kg p.o. sowie anhand der Forsmann-Reaktion im Dosisbereich von etwa 100 bis 300 mg/kg p.o., am Kaninchen anhand ihrer Hemmwirkung auf die durch Arachidonsäure induzierten Lungenembolie im Dosisbereich von etwa 100 bis 300 mg/kg p.o.oder *in vitro* anhand ihrer Prostaglandinsynthesehemmung aus Arachidonsäure durch Rindersamenblasenenzyme im Konzentrationsbereich von etwa 0,1 bis 1 mg/Liter dokumentiert werden. Die Verbindungen der Formel I sind weiterhin anti-inflammatorisch wirksam, was sich z.B. an der Ratte anhand des Kaolin-Pfotenoedems und der Adjuvans-Arthritis im Dosisbereich von etwa 10 bis 100 mg/kg p.o. zeigen lässt. Die neuen Verbindungen sind ferner antinoceptiv wirksam, was sich z.B. anhand des Phenyl-p-benzochinon-writhingsyndroms der Maus im Dosisbereich von etwa 1 bis 10 mg/kg p.o. und des Essigsäure-Writhingsyndroms der Ratte im Dosisbereich von etwa 1 bis 10 mg/kg p.o. zeigen lässt. Sie sind schliesslich uricosurisch wirksam, was sich anhand der Phenolrot-Clearance der Ratte im Dosisbereich von etwa 30 bis 100 mg/kg p.o. nachweisen lässt.

Die Verbindungen der Formel I sind dementsprechend ausgezeichnet geeignet zur Behandlung thrombotischer Erkrankungen, ferner entzündlicher Erkrankungen, insbesondere mit inflammatorischen Komponente, und können als Wirkstoff in antithrombotischen und/oder antiinflammatorischen Arzneimitteln bzw. als Antinoceptiv und/oder Uricosurika verwendet werden.

Die vorliegende Erfindung betrifft dementsprechend auch pharmazeutische Präparate, welche eine der erfindungsgemässen Verbindungen der Formel I bzw. III oder ein pharmazeutisch verwendbares Salz davon enthalten. Bei den erfindungsgemässen pharmazeutischen Präparaten handelt es sich um solche, die zur topischen und lokalen sowie enteralen, wie oralen oder rektalen, sowie parenteralen Verabreichung an und zur Inhalation durch Warmblüter, bestimmt sind und den pharmakologischen Wirkstoff allein oder zusammen mit einem pharmazeutisch anwendbaren Trägermaterial enthalten. Die Dosierung des Wirkstoffs hängt von der Warmblüter-Spezies, dem Alter und dem individuellen Zustand, sowie von der Applikationsweise ab.

Die neuen pharmazeutischen Präparate enthalten z.B. von etwa 10% bis etwa 95%, vorzugsweise von etwa 20% bis etwa 90% des Wirkstoffs. Erfindungsgemässe pharmazeutische Präparate sind z.B. solche in Dosiseinheitsformen, wie Dragées, Tabletten, Kapseln oder Suppositorien, ferner Ampullen.

Die pharmazeutischen Präparaten der vorliegenden Erfindung werden in an sich bekannter Weise, z.B. mittels konventioneller Misch-, Granulier-, Dragier-, Lösungs- oder Lyophilisierungsverfahren hergestellt.

So kann man pharmazeutische Präparate zur oralen Anwendung erhalten, indem man den Wirkstoff mit festen Trägerstoffen kombiniert, ein erhaltenes Gemisch gegebenenfalls granuliert, und das Gemisch bzw. Granulat, wenn erwünscht oder notwendig, nach Zugabe von geeigneten Hilfsstoffen, zu Tabletten oder Dragée-Kernen verarbeitet. Geeignete Trägerstoffe sind insbesondere Füllstoffe, wie Zucker, z.B. Lactose, Saccharose, Mannit oder Sorbit, Cellulosepräparate und/oder Calciumphosphate, z.B. Tricalciumphosphat oder Calciumhydrogenphosphat, ferner Bindemittel, wie Stärkekleister, z.B. von

Mais-, Weizen-, Reis- oder Kartoffelstärkekleister, Gelatine, Tragacanth, Methylcellulose und/oder Polyvinylpyrrolidon, und/oder, wenn erwünscht, Sprengmittel, wie die obgenannten Stärken, ferner Carboxymethylstärke, quervernetztes Polyvinylpyrrolidon, Agar, Alginsäure oder ein Salz davon, wie Natriumalginat. Hilfsmittel sind in erster Linie Fliessregulier- und Schmiermittel, z.B. Kieselsäure, Talk, Stearinsäure oder Salze davon, wie Magnesium- oder Calciumstearat, und/oder Polyäthylenglykol. Dragée-Kerne werden mit geeigneten, gegebenenfalls Magensaft-resistenten Ueberzügen versehen, wobei man u.a. konzentrierte Zuckerlösungen, welche gegebenenfalls arabischen Gummi, Talk, Polyvinylpyrrolidon, Polyäthylenglycol und/oder Titandioxid enthalten, Lacklösungen in geeigneten organischen Lösungsmitteln oder Lösungsmittelgemischen oder, zur Herstellung von Magensaft-resistenten Ueberzügen, Lösungen von geeigneten Cellulosepräparaten, wie Acetylcellulosephthalat oder Hydroxypropylmethylcellulosephthalat, verwendet. Den Tabletten oder Dragée-Ueberzügen können Farbstoffe oder Pigmente, z.B. zur Identifizierung oder zur Kennzeichnung verschiedener Wirkstoffdosen, beigefügt werden.

Weitere oral anwendbare pharmazeutische Präparate sind Steckkapseln aus Gelatine, sowie weiche, geschlossene Kapseln aus Gelatine und einem Weichmacher, wie Glycerin oder Sorbitol. Die Steckkapseln können den Wirkstoff in Form eines Granulats, z.B. im Gemisch mit Füllstoffen, wie Lactose, Bindemitteln, wie Stärken, und/oder Gleitmitteln, wie Talk oder Magnesiumstearat, und gegebenenfalls Stabilisatoren, enthalten. In weichen Kapseln ist der Wirkstoff vorzugsweise in geeigneten Flüssigkeiten, wie fetten Oelen, Paraffinöl oder flüssigen Polyäthylenglykolen, gelöst oder suspendiert, wobei ebenfalls Stabilisatoren zugefügt sein können.

Als rektal anwendbare pharmazeutische Präparate kommen z.B. Suppositorien in Betracht, welche aus einer Kombination des Wirkstoffs mit einer Suppositoriengrundmasse bestehen. Als Suppositoriengrundmasse eignen sich z.B. natürliche oder synthetische Triglyceride, Paraffinkohlenwasserstoffe, Polyäthylenglykole oder höhere Alkanole. Ferner können auch Gelatine-Rektalkapseln verwendet werden, die eine Kombination des Wirkstoffs mit einer Grundmasse enthalten; als Grundmassenstoffe kommen z.B. flüssige Triglyceride, Polyäthylenglykole oder Paraffinkohlenwasserstoffe in Frage.

Zur parenteralen Verabreichung eignen sich in erster Linie wässrige Lösungen eines Wirkstoffs in wasserlöslicher Form, z.B. eines wasserlöslichen Salzes, ferner Suspensionen des Wirkstoffs, wie entsprechende ölige Injektionssuspensionen, wobei man geeignete liphophile Lösungsmittel oder Vehikel, wie fette Oele, z.B. Sesamöl, oder synthetische Fettsäureester, z.B. Aethyloleat oder Triglyceride, verwendet, oder wässrige Injektionssuspensionen, welche viskositätserhöhende Stoffe, z.B. Natriumcarboxymethylcellulose, Sorbit und/oder Dextran und gegebenenfalls auch Stabilisatoren enthalten.

Pharmazeutische Präparate für topische und lokale Verwendung sind z.B. für die Behandlung der Haut Lotionen und Creme, die eine flüssige oder halbfeste Oel-in-Wasser- oder Wasser-in-Oel-Emulsion enthalten, und Salben, die vorzugsweise ein Konservierungsmittel enthalten.

Die Erfindung betrifft ebenfalls die Verwendung der neuen Verbindungen der Formel I und ihrer Salze als Arzneimittel, insbesondere als Antithrombotika, vorzugsweise in der Form von pharmazeutischen Präparaten.

Die nachfolgenden Beispiele illustrieren die oben beschriebene Erfindung; sie sollen diese jedoch in ihrem Umfang in keiner Weise einschränken. Temperaturen werden in Celsiusgraden angegeben.

Beispiel 1

8 g 4-(p-Aethylphenyl)-piperidin, 7,3 g 3,4-Dichlorbenzylchlorid und 16 g wasserfreies Kaliumcarbonat werden unter Rühren in 300 ml Aethanol suspendiert und 5 Stunden unter Rühren zum Rückfluss erhitzt. Man lässt etwas abkühlen, filtriert vom Unlöslichen ab und dampft das Filtrat unter vermindertem Druck zur Trockne ein. Der Rückstand wird mit 200 ml Wasser versetzt und zweimal mit je 150 ml Diäthyläther ausgezogen. Die Aetherextrakte werden vereinigt, mit 100 ml 2n-Natronlauge und anschliessend mit 100 ml gesättigter Kochsalzlösung gewaschen, über Natriumsulfat getrocknet und eingedampft, wobei das rohe 1-(3,4-Dichlorbenzyl)-4-(p-äthylphenyl)-piperidin zurückbleibt. Dieses wird in 100 ml Aethanol gelöst und die Lösung mit äthanolischer Salzsäure (2n) auf pH = 1 angesäuert. Das ausgefallene 1-(3,4-Dichlorbenzyl)-4-(p-äthylphenyl)-piperidin-hydrochlorid wird abgesaugt, mit Diäthyläther gewaschen und getrocknet. Es schmilzt bei 255-257°.

Das Ausgangsmaterial kann z.B. folgendermassen hergestellt werden:

Eine Lösung von 12,0 g α-[p-(4-Piperidyl)-phenyl]-äthanol in 120 ml Eisessig wird mit 1,2 g Palladium (5%-ig auf Kohle) versetzt und bei 40-50° bis zur Aufnahme von 1 Aequivalent Wasserstoff bei Normaldruck hydriert. Dann filtriert man den Katalysator ab und dampft das Filtrat im Vakuum zur Trockne ein. Das im Eindampfrückstand zurückbleibende rohe 4-(4-Aethylphenyl)-piperidin kann durch Behandeln mit Aktivkohle und anschliessende Ueberführung in das Hydrochlorid weitergereinigt werden. Der Schmelzpunkt des Hydrochlorids beträgt 198-202° (aus Aethanol-Aether).

Beispiel 2

In analoger Weise wie in Beispiel 1 beschrieben erhält man durch Umsetzung von 4-(p-Aethylphenyl)-piperidin

mit 2,4-Dichlorbenzylchlorid das 1-(2,4-Dichlorbenzyl)-4-(p-äthylphenyl)-piperidin-hydrochlorid vom Smp. 230°,

mit o-Chlorbenzylchlorid das 1-(o-Chlorbenzyl)-4-(p-äthylphenyl)-piperidin-hydrochlorid vom Smp. 195-196°,

mit p-Chlorbenzylchlorid das 1-(p-Chlorbenzyl)-4-(p-äthylphenyl)-piperidin-hydrochlorid vom Smp. 263-265°,

mit m-Chlorbenzylchlorid das 1-(m-Chlorbenzyl)-4-(p-äthylphenyl)-piperidin-hydrochlorid vom Smp. 230°, und

mit m-Methoxybenzylchlorid das 1-(m-Methoxybenzyl)-4-(p-äthylphenyl)-piperidin-hydrochlorid vom Smp. 157-158°.

Beispiel 3

8 g 4-(p-Acetylphenyl)-piperidin werden in analoger Weise wie in Beispiel 1 beschrieben mit o-Chlorbenzylchlorid zu 1-(o-Chlorbenzyl)-4-(p-acetylphenyl)-piperidin vom Smp. 96-97° umgesetzt.

Beispiel 4

Durch Umsetzung von 5 g 4-[p-(1-Hydroxyäthyl)-phenyl]-piperidin mit o-Chlorbenzylchlorid in der in Beispiel 1 beschriebenen Weise erhält man das 1-(o-Chlorbenzyl)-4-[p-(1-hydroxyäthyl)-phenyl]-piperidin-hydrochlorid vom Smp. 178-179°.

Beispiel 5

Tabletten enthaltend 100 mg Wirkstoff, z.B. 1-(3,4-Dichlorbenzyl)-4-(p-äthylphenyl)-piperidin-hydrochlorid, können beispielsweise in folgender Zusammen-

setzung hergestellt werden:

| Zusammensetzung | Pro Tablette |
|---|---|
| Wirkstoff z.B. 1-(3,4-Dichlorbenzyl)-4-(p-äthylphenyl)-piperidin-hydrochlorid | 100 mg |
| Milchzucker | 50 mg |
| Weizenstärke | 73 mg |
| Kolloidale Kieselsäure | 13 mg |
| Talk | 12 mg |
| Magnesium | 2 mg |
| | 250 mg |

Herstellung

Der Wirkstoff wird mit dem Milchzucker, einem Teil der Weizenstärke und mit kolloidaler Kieselsäure gemischtund die Mischung durch ein Sieb getrieben. Ein weiterer Teil der Weizenstärke wird mit der 5-fachen Menge Wasser auf dem Wasserbad verkleistert und die Pulvermischung mit diesem Kleister angeknetet, bis eine schwach plastische Masse entstanden ist. Die Masse wird durch ein Sieb von ca. 3 mm Maschenweite getrieben, getrocknet und das trockene Granulat nochmals durch ein Sieb getrieben. Darauf werden die restliche Weizenstärke, Talk und Magnesiumstearat zugemischt. Die erhaltene Mischung wird zu Tabletten von 250 mg mit Bruchkerbe(n) verpresst.

Beispiel 6

Eine zum Rückfluss erhitzte Suspension von 1,2 g Lithiumaluminiumhydrid in 50 ml absolutem Tetrahydrofuran wird langsam mit einer Lösung von 19,7 g N-(3,4-Dimethoxybenzoyl)-4-(4-äthylphenyl)-piperidin in 50 ml absolutem Tetrahydrofuran versetzt und weitere 24 Stunden zum Rückfluss erhitzt. Nach dem Abkühlen des Reaktionsgemisches versetzt man mit 10 ml Essigester und wenig Wasser, filtriert und zieht das Tetrahydrofuran ab. Der Rückstand wird in

2n-Salzsäure gelöst und mit wenig Aether gewaschen. Die wässrige Phase wird mit 2n-Natronlauge alkalisch gestellt und mit Aether extrahiert. Nach Waschen der Aetherphase mit Wasser, Trocknen über Magnesiumsulfat und Eindampfen erhält man rohes 1-(3,4-Dimethoxybenzyl)-4-(p-äthylphenyl)-piperidin, das aus äthanolischer Lösung durch Zugabe einer äquivalenten Menge Fumarsäure, gelöst in Aethanol, als Furmarat vom Smp. 151-153° kristallisiert.

Das Ausgangsmaterial kann z.B. in analoger Weise wie in Beispiel 1 beschrieben durch Umsetzung von 4-(p-Aethylphenyl)-piperidin mit 3,4-Dimethoxybenzoylchlorid hergestellt werden. Es schmilzt bei 110-112°.

In analoger Weise kann man durch Umsetzung von 4-(p-Aethylphenyl)-piperidin mit 3,4-Dichlorbenzoylchlorid über 1-(3,4-Dichlorbenzoyl)-4-(p-äthylphenyl)-piperidin vom Smp. 77-79° das 1-(3,4-Dichlorbenzyl)-4-(p-äthylphenyl)-piperidin (Smp. des Hydrochlorides 255-257°) herstellen.

<u>Beispiel 7</u>

In analoger Weise wie in Beispiel 1 beschrieben erhält man durch Umsetzung von 4-(o-Aethylphenyl)-piperidin mit 3,4-Dichlorbenzylchlorid das 1-(3,4-Dichlorbenzyl)-4-(o-äthylphenyl)-piperidin-hydrochlorid von Smp. 250°.

Das Ausgangsmaterial kann folgendermassen hergestellt werden:

Zu einer Suspension von 10 g Magnesiumspänen in wenig Tetrahydrofuran tropft man unter Rühren und Wasserausschluss tropfenweise eine Lösung vom 75 g 1-Aethyl-2-brombenzol in 50 ml Tetrahydrofuran so langsam hinzu, dass die Reaktionstemperatur 60° nicht übersteigt. Anschliessend fügt man innerhalb von 45 Minuten eine Lösung von 57 g N-

Benzyl-4-piperidon in 150 ml Tetrahydrofuran hinzu. Nach beendeter Zugabe lässt man 1 Stunde bei 60° weiterrühren. Die auf 0° gekühlte Reaktionslösung wird mit 1000 ml eiskalter, gesättigter Ammonchloridlösung versetzt und zweimal mit je 1000 ml Aether extrahiert. Die organischen Extrakte werden über Natriumsulfat getrocknet und im Vakuum eingedampft. Destillation des Eindampfrückstandes im Hochvakuum liefert in der bei 175-180° (0,05 mm) siedenden Fraktion 52 g des 4-Hydroxy-4-(2-äthyl-phenyl)-N-benzyl-piperidins als farbloses Oel.

Zu einer Lösung des obigen Produktes in 120 ml Eisessig fügt man 40 ml konzentrierte Salzsäure und 30 ml Wasser hinzu und erhitzt 3 Stunden zum Rückfluss. Dann dampft man im Vakuum zur Trockne ein,stellt mit Natronlauge bis pH 14 alkalisch und extrahiert unter Eiskühlung zweimal mit 1000 ml Essigester. Die organischen Phasen werden mit Wasser gewaschen, über Natriumsulfat getrocknet und im Vakuum eingedampft. Man erhält so das rohe, oelige 1-Benzyl-4-(o-äthylphenyl)-1,2,3,6-tetrahydro-pyridin, das direkt weiterverarbeitet wird. (Hydrochlorid: Smp. 205°).

Zu einer Lösung von 43,5 g 1-Benzyl-4-(o-äthyl-phenyl)-1,2,5,6-tetrahydro-pyridin in 450 ml Eisessig fügt man 48,1 ml 3,4 n-äthanolische Salzsäure und 22,5 g Palladium (5%-ig auf Kohle) und hydriert bei 40-45° bis zur Aufnahme von 2 Aequivalenten Wasserstoff. Dann filtriert man vom Katalysator ab, dampft das Filtrat im Vakuum zur Trockne ein, löst den Eindampfrückstand in 300 ml Wasser, stellt mit Natronlauge auf pH 14 und extrahiert zweimal mit 500 ml Aether. Die organischen Extrakte werden über Natriumsulfat getrocknet und im Vakuum zur Trockne eingedampft. Den Eindampfrückstand löst man in 130 ml Aethanol und versetzt bei 60° mit einer heissen Lösung von 15,5 g Fumarsäure in 350 ml Wasser. Das beim Abkühlen kristallisierende 4-(o-Aethylphenyl)-piperidin-fumarat wird abgenutscht, mit Aethanol und Aether gewaschen und im Vakuum bei Raumtemperatur getrocknet. Smp. 195°.

Beispiel 8

In analoger Weise wie in Beispiel 1 beschrieben erhält man durch Umsetzung von 4-(m-Aethylphenyl)-piperidin mit 3,4-Dichlorbenzylchlorid das 1-(3,4-Dichlorbenzyl)-4-(m-äthylphenyl)-piperidin-hydrochlorid vom Smp. 249-50°.

Das Ausgangsmaterial kann folgendermassen hergestellt werden:

Umsetzung von 60 g 3-Aethyl-brombenzol mit Magnesium und 61 g N-Benzyl-4- piperidon ergibt 4-Hydroxy-4-(m-äthylphenyl)-N-benzylpiperidin vom Kp. 195-200° (0,04mm).

Daraus erhält man mit Eisessig/Salzsäure öliges rohes 1-Benzyl-4-(o-äthylphenyl)-1,2,3,6-tetrahydro-pyridin, das ohne weitere Reinigung in analoger Weise wie in Beispiel 7 (Herstellung des Ausgangsmaterials) beschrieben durch Hydrierung mit Palladium (5%-ig auf Kohle) in das 4-(m-Aethylphenyl)-piperidin-fumarat überführt wird.

Beispiel 9

Zu einer Lösung von 3,5 g 3,4-Dichlorbenzylamin in 100 ml Aethanol und 20 ml 2n-Natronlauge gibt man unter Rühren bei 0° eine Lösung von 6,7 g 3-(p-Aethylphenyl)-1,5-dibrom-pentan.Nach beendeter Zugabe wird die Reaktionsmischung 10 Stunden zum Rückfluss erhitzt. Dann kühlt man auf Raumtemperatur, entfärbt mit wenig Natriumthiosulfat, dampft im Vakuum zur Trockne ein und destilliert den Rückstand im Hochvakuum. Die bei 90-140° (0,03 mmHg) siedende

Fraktion wird durch Chromatographie an Kieselgel mit Chloroform/Methanol (15:1) weiter gereinigt. Man erhält das 1-(3,4-Dichlorbenzyl)-4-(p-äthylphenyl)-piperidin. Dieses wird zur Charakterisierung wie in Beispiel 1 beschrieben in das Hydrochlorid vom Smp. 255-258° überführt. Das 1-(3,4-Dichlorbenzyl)-4-(p-äthylphenyl)-piperidin-fumarat kann analog hergestellt werden. Es schmilzt bei 255-257°.

Das als Ausgangsmaterial verwendete 3-(p-Aethylphenyl)-1,5-dibrom-pentan lässt sich z.B. folgendermassen herstellen:

Eine Lösung von 43 g p-Aethylbenzaldehyd und 52 g Diäthylphosphoroacetonitril in 300 ml Methylenchlorid wird innerhalb von 15 Minuten unter Eiskühlung zu einer gut gerührten Emulsion von 6,5 g Tetrabutylammoniumbromid in 180 ml 50%-iger Natronlauge und 150 ml Methylenchlorid zugetropft. Anschliessend rührt man 30 Minuten bei Raumtemperatur nach. Dann wird die organische Phase abgetrennt, mit Wasser neutralgewaschen, über Natriumsulfat getrocknet und im Vakuum eingedampft. Das so erhaltene rohe p-Aethylzimtsäurenitril [Oel; JR ($CH_2Cl_2$): $\nu$max 2240 $cm^{-1}$] wird zu einer Lösung von 8 g Natrium in 53 g Malonsäurediäthylester und 400 ml absolutem Aethanol gegeben und 2 Stunden zum Rückfluss erhitzt. Dann dampft man in Vakuum auf einen Drittel des Volumens ein, versetzt mit 500 ml wässriger Essigsäure (0,5n) und extrahiert dreimal mit 500 ml Aether. Die organischen Phasen wäscht man neutral, trocknet über Natriumsulfat und dampft im Vakuum zur Trockne ein. Chromatographie des Eindampfrückstandes an 1 kg Kieselgel mit Methylenchlorid als Laufmittel liefert das 2-Carboäthoxy-3-(p-äthylphenyl)-propan-1-carbonsäureäthylester-3-nitril als farbloses Oel [IR ($CH_2Cl_2$) $\nu$max: 1720 $cm^{-1}$ (Schulter)].

Das 2-Carboäthoxy-3-(p-äthylphenyl)-propan-1-carbonsäureäthylester-3-nitril lässt sich analog zu dem im J. Amer. Chem. Soc,; 53, 1105 (1931) beschriebenen Verfahren zum 3-(p-Aethylphenyl)-1,5-dibrom-pentan umsetzen. Dieses siedet bei 175-185° (12 mmHg).

Beispiel 10

Zu einer Suspension von 150 mg Lithiumaluminiumhydrid in 20 ml absolutem Tetrahydrofuran fügt man unter Rühren in einer Stickstoffatmosphäre portionsweise 1 g 1-(3,4-Dimethoxybenzyl)-4-(p-äthylphenyl)-2-piperidon hinzu. Dann lässt man 1 Stunde bei 60° nachrühren, kühlt auf Raumtemperatur ab, versetzt tropfenweise mit 0,5 ml Wasser und 0,1 ml 2n-Natronlauge und filtriert über Kieselgur. Das Filtrat wird eingedampft und das im Eindampfrückstand verbliebene rohe 1-(3,4-Dimethoxybenzyl)-4-(4-äthylphenyl)-piperidin wie in Beispiel 6 beschrieben in das Fumarat vom F 151-153° überführt.

Das Ausgangsmaterial kann wie folgt erhalten werden:

Eine Lösung von 31 g 2-Carboäthoxy-3-(p-äthylphenyl)-propan-1-carbonsäureäthylester-3-nitril in 1,6 g Triäthylamin und 500 ml absolutem Aethanol wird mit 8 g Raney-Nickel bis zur Aufnahme von etwa 4 l Wasserstoff hydriert. Dann filtriert man vom Katalysator ab und dampft im Vakuum zur Trockne ein. Aus dem Eindampfrückstand kristallisiert mit Aether das 3-Carboäthoxy-4-(p-äthylphenyl)-2-piperidon vom F -157-159°.

Eine Lösung von 8 g der obigen Verbindung in 110 ml Aethanol, 40 ml 2n-Natronlauge und 50 ml Wasser wird während 45 Minuten am Rückfluss gekocht. Dann dampft man

im Vakuum zur Trockne ein, stellt den Eindampfungsrückstand mit 2n-Salzsäure auf pH 1 und extrahiert mit 500 ml Chloroform. Die organische Phase wird neutral gewaschen, über Natriumsulfat getrocknet und im Vakuum eingedampft. Das im Eindampfrückstand verbleibende rohe, kristalline 3-Carboxy-4-(4-äthylphenyl)-2-piperidon (Smp. 125° Zers.) wird in 400 ml Toluol 30 Minuten zum Rückfluss erhitzt. Dann dampft man auf ein Volumen vom etwa 20 ml ein und versetzt mit wenig Aether. Dabei kristallisiert das 4-(p-Aethylphenyl)-2-piperidon vom F 165° aus.

Zu einer Lösung vom 2 g 4-(p-Aethylphenyl)-2-piperidon und 3,2 g Tetrabutylammoniumbromid in 40 ml Methylenchlorid gibt man 2,3 g 3,4-Dimethoxybenzylchlorid und 40 ml 30%-iger Natronlauge. Dieses Gemisch wird 4 Stunden gerührt und anschliessend von der wässrigen Phase abgetrennt. Die organische Phase wird mit Wasser neutral gewaschen, über Natriumsulfat getrocknet und eingedampft. Der Eindampfrückstand wird mit Aether verrührt. Der ätherlösliche Anteil, der das 1-(3,4-Dimethoxybenzyl)-4-(p-äthylphenyl)-2-piperidon enthält, wird eingedampft und ohne weitere Reinigung in der nächsten Stufe eingesetzt.

Beispiel 11

Eine Lösung von 6,4 g 1-(m-Methoxybenzyl)-4-(p-acetylphenyl)-piperidin und 5 ml Hydrazinhydrat in 50 ml Aethylenglykol wird mit 7,5 g pulverisiertem Natriumhydroxid versetzt und 1,5 Stunden am Rückfluss gekocht. Anschliessend destilliert man ein Gemisch von Hydrazin und Wasser ab, bis die Sumpftemperatur 190° erreicht. Nach dem Abkühlen des Reaktionsgemisches wird mit der gleichen Menge Wasser versetzt und mit Aether extrahiert. Die Aetherphase wird über Natriumsulfat getrocknet und eingedampft.

Der Eindampfrückstand wird an Kieselgel mit Methylenchlorid/Methanol (15:1) als Eluens chromatographiert und liefert so das 1-(m-Methoxybenzyl)-4-(4-äthylphenyl)-piperidin, das auf übliche Weise in das Hydrochlorid vom Smp. 157-158° überführt wird.

Beispiel 12

In analoger Weise wie in Beispiel 1 beschrieben erhält man durch Umsetzung von 4-(p-Aethylphenyl)-piperidin mit p-Fluorbenzylchlorid das 1-(p-Fluorbenzyl)-4-(p-äthylphenyl)-piperidin-hydrochlorid vom Smp. 250-52° und mit Benzylchlorid das 1-Benzyl-4-(p-äthylphenyl)-piperidin-fumarat vom Smp. 183-185°.

Beispiel 13

In analoger Weise wie in Beispiel 1 bzw. 3 beschrieben erhält man durch Umsetzung von 4-(p-Acetylphenyl)-piperidin
mit m-Methoxybenzylbromid das 1-(m-Methoxybenzyl)-4-(p-acetylphenyl)-piperidin-fumarat vom Smp. 157-59°,
mit 3,4-Dichlorbenzylchlorid das 1-(3,4-Dichlorbenzyl)-4-(p-acetylphenyl)-piperidin-hydrochlorid vom Smp. 258-60° und
mit Benzylchlorid das 1-Benzyl-4-(p-acetylphenyl)-piperidin-fumarat vom Smp. 161-62°.

Beispiel 14

In analoger Weise wie in Beispiel 4 beschrieben erhält man durch Umsetzung von 4-[p-(1-Hydroxyäthyl)-phenyl]-piperidin mit Benzylchlorid das 1-Benzyl-4[p-(1-hydroxyäthyl)-phenyl]-piperidin-fumarat vom Smp. 154-158°,
mit m-Methoxybenzylbromid das 1-(m-Methoxybenzyl)-4-[p-

(l-hydroxyäthyl)-phenyl]-piperidin-fumarat vom Smp. 160-61° und

mit 3,4-Dichlorbenzylchlorid das 1-(3,4-Dichlorbenzyl)-4-[p-(1-hydroxyäthyl)-phenyl]-piperidin-fumarat vom Smp. 195-97°.

Beispiel 15

8 g 4-(p-Aethylphenyl)-piperidin, 4,2 g Benzylchlorid und 16 g wasserfreies Kaliumcarbonat werden unter Rühren in 300 ml Aethanol suspendiert und 5 Stunden zum Rückfluss erhitzt. Man lässt etwas abkühlen, saugt vom Unlöslichen ab und dampft unter vermindertem Druck zur Trockne ein. Der Rückstand wird mit 200 ml Wasser versetzt und zweimal mit je 170 ml Diäthyläther ausgezogen. Die Aetherextrakte werden vereinigt, mit 100 ml 2n-Natronlauge und anschliessend mit 100 ml gesättigter Kochsalzlösung gewaschen, über Natriumsulfat getrocknet und zur Trockne eingedampft. Man erhält 1-Benzyl-4-(p-äthylphenyl)-piperidin, das durch Auflösen in Aethanol und Ansäuern mit äthanolischer Salzsäure in das 1-Benzyl-4-(p-äthylphenyl)-piperidin-hydrochlorid überführt werden kann.

Das Ausgangsmaterial kann z.B. folgendermassen erhalten werden:

Eine Lösung von 12,0 g α-[p-(4-Piperidyl)-phenyl]-äthanol in 120 ml Eisessig wird mit 1,2 g Palladium (5%-ig auf Kohle) versetzt und bei 40-50° bis zur Aufnahme von 1 Aequivalent Wasserstoff bei Normaldruck hydriert. Dann filtriert man den Katalysator ab und dampft das Filtrat im Vakuum zur Trockne ein. Das im Eindampfrückstand zurückbleibende rohe 4-(4-Aethylphenyl)-piperidin kann durch Behandeln mit Aktivkohle und anschliessende Ueberführung in das Hydrochlorid weitergereinigt werden. Der Schmelzpunkt des Hydrochlorids beträgt 198-202° (aus Aethanol-Aether).

Beispiel 16

In analoger Weise wie in Beispiel 15 beschrieben kann 1-Benzyl-4-(2,4-dimethyl-phenyl)-1,2,5,6-tetrahydro-pyridin durch Ueberführung von 34 g 4-Brom-m-xylol in die Bromomagnesiumverbindung nach Grignard, Umsetzung derselben mit 38 g N-Benzyl-4-piperidon und anschliessende Wasserabspaltung mittels Essigsäure-Salzsäure erhalten werden, das unter 0,05 mmHg bei etwa 170° siedet, ebenso 1-Benzyl-1,2,5,6-tetrahydro-4-(p-methylphenyl)-piperidin, Smp. 40-41° (aus kaltem Pentan).

Beispiel 17

Eine Lösung von 55 g 1-Benzyl-4-hydroxy-4-[p-(1-äthylendioxyäthyl)-phenyl]-piperidin in 250 ml konzentrierter Salzsäure wird drei Stunden zum Rückfluss erhitzt. Dann dampft man im Vakuum zur Trockne ein, stellt mit 2n-Natronlauge auf pH 14 ein und extrahiert dreimal mit je 500 ml Diäthyläther. Die organischen Phasen werden vereinigt, neutralgewaschen, über Natriumsulfat getrocknet und auf ein Drittel eingedampft. Es kristallisiert reines 1-Benzyl-1,2,5,6-tetrahydro-4-(4-acetylphenyl)-pyridin vom Smp. 99-100° aus.

Das Ausgangsmaterial kann z.B. folgendermassen hergestellt werden:

Zu einer Suspension von 9,8 g Magnesiumspänen in 100 ml absolutem Tetrahydrofuran tropft man bei etwa 50° unter Wasserausschluss langsam eine Lösung vom 97 g 4-Brom-acetophenon-äthylenketal in 100 ml absolutem Tetrahydrofuran hinzu. Sobald alles Magnesium in Lösung gegangen ist, kühlt man auf 5° ab und versetzt tropfenweise mit einer Lösung von 70 g N-Benzyl-4-piperidon in 100 ml

absolutem Tetrahydrofuran. Nach beendeter Zugabe wird im Vakuum zur Trockne eingedampft und der Eindampfrückstand mit wasserfreien Aethyläther verrieben und abgenutscht. Das Nutschgut wird anschliessend zwischen 3-mal 200 ml Aether und 200 ml gesättigter, kalter wässriger Ammoniumchloridlösung verteilt. Die organischen Phasen werden vereinigt, neutralgewaschen, über Natriumsulfat getrocknet und im Vakuum zur Trockne eingedampft. Das so erhaltene 1-Benzyl-4-hydroxy-4-[p-(1-äthylendioxyäthyl)-phenyl]-piperidin schmilzt bei 114-116°.

Beispiel 18

Zu einer Suspension von 0,5 g Magnesiumspänen, überschichtet mit wenig absolutem Diäthyläther fügt man unter Stickstoff einige Tropfen Methyljodid und nach Anspringen der Reaktion bei 30-35° tropfenweise eine Lösung von 6,3 g 1-(m-Methoxybenzyl)-4-(p-chlorphenyl)-piperidin in 20 ml absolutem Diäthyläther hinzu. Nachdem der grösste Teil des Magnesiums gelöst ist, versetzt man mit 100 mg Kupfer-I-jodid, kühlt auf -10° ab, fügt 2,5 g Aethylbromid hinzu und lässt über Nacht bei -10 bis 0° rühren. Dann wird mit 50 ml 2n-Natronlauge versetzt, mit Kochsalz gesättigt und dreimal mit je 50 ml Diäthyläther extrahiert. Die organischen Phasen werden neutralgewaschen, über Natriumsulfat getrocknet und im Vakuum eingedampft. Destillation des Eindampfrückstandes im Hochvakuum und Chromatographie der bei 100-150° (0,03 mmHg) siedenden Fraktion an Kieselgel mit Chloroform und wenig Methanol als Laufmittel ergibt rohes 1-(m-Methoxybenzyl)-4-(p-äthylphenyl)-piperidin (Smp. des Hydrochlorides: 157-158°).

Das Ausgangsmaterial kann z.B. folgendermassen hergestellt werden:

Behandlung von p-Chlorzimmtsäureäthylester mit Natriumdiäthylmalonat zum ß-Phenyl-α,α,γ-tricarbonsäuretriäthylester, anschliessender Verseifung mit Kalilauge und Decarboxylierung bei 110-130° führt zur ß-p-Chlorphenylglutarsäure, welche durch Umsetzung mit m-Methoxybenzylamin in das 1-(m-Methoxybenzyl)-4-(p-chlorphenyl)-piperidin-2,6-dion überführt wird. Dieses wird dann mit Lithiumaluminiumhydrid zum gewünschten 1-(m-Methoxybenzyl)-4-(4-chlorphenyl)-piperidin reduziert.

## Beispiel 19

Tabletten enthaltend 100 mg Wirkstoff, z.B. 1-Benzyl-4-(p-äthylphenyl)-piperidin, oder dessen Hydrochlorid, können beispielsweise in folgender Zusammensetzung hergestellt werden:

| Zusammensetzung | pro Tablette |
|---|---|
| Wirkstoff, z.B. 1-Benzyl-4-(p-äthylphenyl)-piperidin | 100 mg |
| Milchzucker | 50 mg |
| Weizenstärke | 73 mg |
| Kolloidale Kieselsäure | 13 mg |
| Talk | 12 mg |
| Magnesiumstearat | 2 mg |
| | 250 mg |

### Herstellung

Der Wirkstoff wird mit dem Milchzucker, einem Teil der Weizenstärke und mit kolloidaler Kieselsäure gemischt und die Mischung durch ein Sieb getrieben. Ein weiterer Teil der Weizenstärke wird mit der 5-fachen Menge Wasser auf dem Wasserbad verkleistert und die Pulvermischung mit diesem Kleister angeknetet, bis eine schwach plastische Masse entstanden ist. Die Masse wird durch ein

Sieb von ca. 3 mm Maschenweite getrieben, getrocknet und das trockene Granulat nochmals durch ein Sieb getrieben. Darauf werden die restliche Weizenstärke, Talk und Magnesiumstearat zugemischt. Die erhaltene Mischung wird zu Tabletten von 250 mg mit Bruchtkerbe(n) verpresst.

Beispiel 20

In analoger Weise wie in den Beispielen 5 und 19 beschrieben kann man auch Tabletten enthaltend eine andere Verbindung gemäss einem der Beispiele 1-4 und 6-18 herstellen.

## Patentansprüche

1. Neue substituierte, gegebenenfalls einfach ungesättigte 1-Benzyl-phenylpiperidine der Formel

$$R_1 - Ph - R_2 \qquad (I),$$

worin $R_1$ einen in 1-Stellung durch gegebenenfalls substituiertes Benzyl substituierten, gegebenenfalls einfach ungesättigten Piperidylrest bedeutet, Ph einen gegebenenfalls substituierten Phenylenrest darstellt und $R_2$ einen gegebenenfalls substituierten Niederalkylrest bedeutet, mit der Massgabe, dass Ph gegebenenfalls substituiertes 1,2- oder 1,3-Phenylen bedeutet, wenn $R_2$ in α-Stellung eine Hydroxy-, Hydroxymethyl-, Niederalkanoyl-, 2-Oxoniederalkanoyl- oder 2-Hydroxyniederalkanoylgruppe aufweist, und mit der weiteren Massgabe, dass $R_1$ von 4-(1-Benzyl)-piperidyl und 4-(1-Benzyl)-, 4-[1-(Monohalogenbenzyl)]- und 4-[1-(Monoalkylbenzyl)]-1,2,5,6-tetrahydro-pyridyl verschieden ist, wenn Ph gegebenenfalls substituiertes 1,4-Phenylen darstellt und $R_2$ Niederalkyl oder 1-Hydroxyniederalkyl oder Niederalkanoyl mit mindestens 2 C-Atomen ist, und ihre Salze.

2. Verbindungen gemäss Anspruch 1, worin $R_1$ einen im Phenylteil gegebenenfalls durch Niederalkyl mit bis und mit 4 C-Atomen, Niederalkoxy mit bis und mit 4 C-Atomen, Hydroxy, Halogen mit Atomnummer bis und mit 35, Hydroxy und/oder Trifluormethyl substituiertes 3- oder 4-(1-Benzyl)-piperidyl- bzw. 3- oder 4-(1-Benzyl-1,2,5,6-tetrahydro)-pyridylrest bedeutet, Ph gegebenenfalls durch Niederalkyl mit bis und mit 4 C-Atomen, Niederalkoxy mit bis und mit 4 C-Atomen, Hydroxy oder Halogen mit Atomnummer bis und mit 35 substituiertes 1,4- oder 1,3-Phenylen dar-

stellt, und $R_2$ gegebenenfalls in höherer als der 2-Stellung durch Hydroxy oder in 1-Stellung durch Oxo substituiertes Niederalkyl mit bis und mit 4 C-Atomen bedeutet, mit der Massgabe, dass $R_1$ von unsubstituiertem 4-(1-Benzyl)-piperidyl und gegebenenfalls durch Niederalkyl oder Halogen monosubstituiertem 4-(1-Benzyl-1,2,5,6-tetrahydropyridyl oder Ph von gegebenenfalls substituiertem 1,4-Phenylen verschieden ist, wenn $R_2$ Niederalkyl oder Niederalkanoyl mit mindestens 2 C-Atomen bedeutet, und ihre Salze.

3. Verbindungen gemäss Anspruch 1, worin $R_1$ im Phenylteil durch Niederalkoxy mit bis und mit 4 C-Atomen und/oder Halogen mit Atomnummer bis und mit 35, mono- oder disubstituiertes 3- oder 4-(1-Benzyl)-piperidyl bedeutet, Ph 1,4-Phenylen bedeutet und $R_2$ gegebenenfalls in 1-Stellung oder in höherer als der 2-Stellung durch Oxo oder in höherer als der 2-Stellung durch Hydroxy monosubstituiertes Niederalkyl mit bis und mit 4 C-Atomen bedeutet, und ihre Salze.

4. Verbindungen gemäss Anspruch 1, worin $R_1$ im Phenylteil durch Niederalkoxy mit bis und mit 4 C-Atomen monosubstituiertes oder durch Halogen bis und mit Atomnummer 35 disubstituiertes 4-(1-Benzyl)-piperidyl bedeutet, Ph 1,4-Phenylen darstellt und $R_2$ Niederalkyl mit bis und mit 4 C-Atomen bedeutet,und ihre Salze.

5. 1-(3,4-Dichlorbenzyl)-4-(p-äthylphenyl)-piperidin oder ein Salz davon.

6. 1-(2,4-Dichlorbenzyl)-4-(p-äthylphenyl)-piperidin oder ein Salz davon.

7. 1-(3,4-Dimethoxybenzyl)-4-(p-äthylphenyl)-piperidin oder ein Salz davon.

8. Verfahren zur Herstellung neuer substituierter, gegebenenfalls einfach ungesättigter 1-Benzyl-phenylpiperidine der Formel

$$R_1 - Ph - R_2 \qquad (I),$$

worin $R_1$ einen in 1-Stellung durch gegebenenfalls substituiertes Benzyl substituierten, gegebenenfalls einfach ungesättigten Piperidylrest bedeutet, Ph einen gegebenenfalls substituierten Phenylenrest darstellt und $R_2$ einen gegebenenfalls substituierten Niederalkylrest bedeutet, mit der Massgabe, dass Ph gegebenenfalls substituiertes 1,2- oder 1,3-Phenylen bedeutet, wenn $R_2$ in α-Stellung eine Hydroxy-, Hydroxymethyl-, Niederalkanoyl-, 2-Oxoniederalkanoyl- oder 2-Hydroxyniederalkanoylgruppe aufweist, und mit der weiteren Massgabe, dass $R_1$ von 4-(1-Benzyl)-piperidyl und 4-(1-Benzyl)-, 4-[1-(Monohalogenbenzyl)- und 4-[1-(Monoalkylbenzyl)]-1,2,5,6-tetrahydro-pyridyl verschieden ist, wenn Ph gegebenenfalls substituiertes 1,4-Phenylen darstellt und $R_2$ Niederalkyl bedeutet oder Niederalkanoyl oder 1-Hydroxyniederalkyl mit mindestens 2 C-Atomen ist, und ihrer Salze, dadurch gekennzeichnet, dass man in einer Verbindung der Formel

$$R_A - Ph - R_2 \qquad (II),$$

worin $R_A$ einen in 5-Stellung durch im Phenylteil gegebenenfalls substituiertes Benzylamino substituierten, in 1-Stellung einen austauschbaren Rest X oder in 1,2-Stellung eine gegebenenfalls zusätzliche Doppelbindung aufweisenden, gegebenenfalls einfach ungesättigten n-Pentylrest bedeutet, oder in einem Salz davon, $R_A$ zu dem entsprechenden Rest $R_1$ ringschliesst oder eine Verbindung der Formel

$$R_B - Ph - R_2 \qquad (III),$$

worin $R_B$ einen 1-unsubstituierten, gegebenenfalls einfach ungesättigten Piperidylrest bedeutet, oder ein Salz davon durch Umsetzung mit einem den gegebenenfalls substituierten Benzylrest einführenden Mittel in 1-Stellung substituiert oder in einer Verbindung der Formel

$$R_C - Ph - R_2 \qquad (IV),$$

worin $R_C$ einen durch mindestens einen durch Wasserstoff ersetzbaren Rest substituierten und/oder mindestens eine gegebenenfalls zusätzliche Doppelbindung aufweisenden Rest $R_1$ bedeutet, oder einem Salz davon den Rest $R_C$ zu $R_1$ reduziert oder in einer entsprechender Verbindung der Formel

$$R_D - Ph - R_2 \qquad (V),$$

worin $R_D$ einen in 1-Stellung durch gegebenenfalls substituiertes Benzyl und durch einen der α,β-Eliminierung zugänglichen Rest substituierten Piperidylrest bedeutet, oder einem Salz davon diesen zusammen mit einem β-ständigen Wasserstoffatom eliminiert oder in einer Verbindung

$$R_1 - Ph - R_E \qquad (VI),$$

worin $R_E$ eine in einen Rest $R_2$ überführbare Gruppe bedeutet, oder in einem Salz davon $R_E$ in $R_2$ überführt oder auf ein Gemisch von Verbindungen der allgemeinen Formel

$$R_o - Ph - H \quad (VII) \quad \text{und} \quad R_o' - X_1 \quad (VIII),$$

worin einer der Reste $R_o$ und $R_o'$ einen Rest $R_1$ und der andere einen Rest $R_2$ bedeutet, und $X_1$ eine gegebenenfalls reaktionsfähig veresterte Hydroxygruppe oder eine sich zu einem benachbarten Kohlenstoffatom erstreckende Bindung bedeutet, ein geeignetes saures Mittel einwirken lässt oder Verbindungen der Formeln

$$R_o - Ph - X_2 \quad (IX) \quad und \quad R''_o \overset{\cdot}{=\!=} X_3 \ (X) ,$$

worin einer der Reste $R_o$ und $R''_o$ einen Rest $R_1$ und der andere einen Rest $R_2$ und einer der Reste $X_2$ und $X_3$ ein Metallradikal und der andere Halogen oder an einen Niederalkylrest $R''_o$ gebundenes Oxo oder an einen Niederalkanoylrest $R''_o$ gebundenes in Salzform vorliegendes, veräthertes oder verestertes Hydroxy bedeutet, miteinander kondensiert und gewünschtenfalls ein erhaltenes Steroisomerengemisch in die Komponenten auftrennt, die erhaltene Verbindung in eine andere Verbindung der Formel I überführt und/oder eine erhaltene freie Verbindung in ein Salz oder ein erhaltenes Salz in die freie Verbindung oder in ein anderes Salz umwandelt.

9. Substituierte, gegebenenfalls einfach ungesättigte 1-Benzyl-phenylpiperidine der Formel

$$R_1 - Ph - R_2 \qquad (I) ,$$

worin $R_1$ einen in 1-Stellung durch gegebenenfalls substituiertes Benzyl substituierten, gegebenenfalls einfach ungesättigten Piperidylrest bedeutet, Ph einen gegebenenfalls subtituierten Phenylenrest darstellt und $R_2$ einen gegebenenfalls substituierten Niederalkylrest bedeutet, und ihre Salze oder Verbindungen gemäss einem der Ansprüche 1-22 und als Arzneimittel, wie Antithrombotikum.

10. Verfahren zur Herstellung von pharmazeutischen Präparaten enthaltend eine Verbindung gemäss einem der Ansprüche 1-7, dadurch gekennzeichnet, dass man diese üblichen pharmazeutischen Träger- und/oder Hilfsstoffen verarbeitet.

Europäisches Patentamt

# EUROPÄISCHER RECHERCHENBERICHT

Nummer der Anmeldung

EP 79 102 272.6

| EINSCHLÄGIGE DOKUMENTE | | | KLASSIFIKATION DER ANMELDUNG (Int.Cl.³) |
|---|---|---|---|
| Kategorie | Kennzeichnung des Dokuments mit Angabe, soweit erforderlich, der maßgeblichen Teile | betrifft Anspruch | |
| | DE - A - 2 101 997 (FARBENFABRIKEN BAYER)<br>* Anspruch 3 *<br>-- | 1-4 | C 07 D 211/14<br>C 07 D 211/22<br>C 07 D 211/32<br>C 07 D 211/70<br>A 61 K 31/445 |
| | DE - A1 - 2 705 416 (ROUSSEL-UCLAF)<br>* Ansprüche 1, 2, 9, 14 bis 19 *<br>-- | 1,2, 9,10 | |
| P | DE - A1 - 2 801 195 (CIBA-GEIGY)<br>* Anspruch 23 *<br>-- | 8 | RECHERCHIERTE SACHGEBIETE (Int. Cl.³) |
| P | FR - A2 - 2 380 261 (ROUSSEL-UCLAF)<br>* Ansprüche 1, 3, 4 *<br>-- | 1,2, 8 | A 61 K 31/445<br>C 07 D 211/14<br>C 07 D 211/22<br>C 07 D 211/32<br>C 07 D 211/70 |
| A | DE - A - 1 620 074 (N.V. KONINKLIJKE PHARMACEUTISCHE FABRIEKEN V/H BROCADES-STHEEMAN & PHARMACIA)<br>-- | | |
| P,A | DE - A1 - 2 839 883 (PFIZER)<br>---- | | KATEGORIE DER GENANNTEN DOKUMENTE<br>X: von besonderer Bedeutung<br>A: technologischer Hintergrund<br>O: nichtschriftliche Offenbarung<br>P: Zwischenliteratur<br>T: der Erfindung zugrunde liegende Theorien oder Grundsätze<br>E: kollidierende Anmeldung<br>D: in der Anmeldung angeführtes Dokument<br>L: aus andern Gründen angeführtes Dokument<br>&: Mitglied der gleichen Patentfamilie, übereinstimmendes Dokument |
| X | Der vorliegende Recherchenbericht wurde für alle Patentansprüche erstellt. | | |

| Recherchenort | Abschlußdatum der Recherche | Prüfer |
|---|---|---|
| Berlin | 31-10-1979 | FROELICH |